# EUROPÄISCHE PATENTSCHRIFT

(11) **EP 0 388 593 B1**
(45) Veröffentlichungstag und Bekanntmachung des Hinweises auf die Patenterteilung: **10.04.1996**
(21) Anmeldenummer: 90101399.5
(22) Anmeldetag: 24.01.1990
(51) Int. Cl.: C12N 15/55, C12N 15/56, C12N 15/80, C12N 9/18, C12N 9/24

(54) **Verfahren zur Expression von Polygalakturonidase und Pektinesterase in Aspergillus niger und Aspergillus awamori**
Method for the expression of polygalacturonidase and pectinesterase in Aspergillus niger and Aspergillus awamori
Méthode par l'expression de polygalacturonidase et pectinestérase dans Aspergillus niger et Aspergillus awamori

(30) Priorität: 17.03.1989 DE 3908813
(43) Veröffentlichungstag der Anmeldung: 26.09.1990
(73) Patentinhaber: RÖHM GMBH Chemische Fabrik, D-64293 Darmstadt (DE)
(72) Erfinder: Ruttkowksi, Edeltraud, Dr., D-6100 Darmstadt (DE); Nguyen, Quoc Khahn, Dipl.Ing.Dr., D-6100 Darmstadt (DE); Gottschalk, Michael, Dipl.Biol.Dr., D-6105 Ober-Ramstadt (DE); Jany, Klaus-Dieter, Prof.Dr., D-7031 Steinenbronn (DE); Löffler, Fridolin, Dipl.Chem.Dr., D-6140 Bensheim (DE); Piepersberg, Wolfgang, Prof.Dr., D-5600 Wuppertal (DE); Schuster, Erwin, Dr., D-6140 Bensheim-Auerbach (DE); Gassen, Hans Günter, Prof.Dr., D-6100 Darmstadt (DE)

(56) Entgegenhaltungen:
- EP-A- 0 222 279
- EP-A- 0 271 988
- EP-A- 0 324 399
- DE-A- 3 044 455
- FR-A- 2 021 772

## Beschreibung

Die Erfindung betrifft die Isolierung und Identifizierung einer Desoxyribonucleinsäure aus Aspergillus niger sowie ein Verfahren zur Expression einer aus Aspergillus stammenden Pektinase in einem Wirtsorganismus.

### Stand der Technik

Ein Verfahren zur Expression eines beispielsweise aus Aspergillus niger stammenden Gens in einem Aspergillus oryzae als Wirtsorganismus ist aus der EP-A 238 023 bekannt. Das Verfahren umfaßt die Schritte:
a) Erstellung eines rekombinanten DNA-Klonierungs-Vektorsystems, das die Fähigkeit zur ein- oder mehrfachen Integration in das Genom eines Aspergillus oryzae als Wirtsorganismus hat, enthaltend DNA-Sequenzen zur Genexpression, ein geeignetes Marker-Gen zur Selektion von Transformanten, sowie eine DNA-Sequenz, die das zu exprimierende Gen darstellt;
b) Transformation des Aspergillus oryzae-Wirtsorganismus, der in Bezug auf das verwendete Marker-Gen selektierbar ist, mit dem gemäß (a) erstellten rekombinanten DNA-Klonierungs-Vektorsystem;
c) Vermehrung des transformierten Aspergillus oryzae-Wirtsorganismus in einem geeigneten Nährmedium.

Nach dem bekannten Verfahren lassen sich Glukamylasen, alpha-Amylasen, Lipasen und Proteinasen erzeugen, die u.a. vom Genom eines Aspergillus niger stammen können. Es wurde jedoch festgestellt, daß bei der Anwendung des Verfahrens zur Erzeugung von Pektinasen, insbesondere vom PE- und PG-Typ, nicht die erhoffte Ausbeuteverbesserung erreicht wird.

### Aufgabe und Lösung

Der Erfindung liegt die Aufgabe zugrunde, ein Verfahren zu finden, mit dem sich auch Pektinasen aus Aspergillus niger vorteilhaft exprimieren lassen. Dazu ist es erforderlich, die Desoxyribonukleinsäuren für die jeweilige Pektinase zu isolieren und zu identifizieren sowie ein Expressionssystem in Gestalt eines rekombinanten Mikroorgamismus zur Gewinnung der Pektinase zu entwickeln. Überraschenderweise gelingt dies erfindungsgemäß durch eine Abwandlung des oben erwähnten bekannten Verfahrens, indem als Wirtsorganismus ein Schimmelpilz der Arten Aspergillus niger oder Aspergillus awamori eingesetzt wird und im übrigen die aus dem Stand der Technik bekannte Arbeitsweise, wie in den Patentansprüchen angegeben, angewendet wird.

Zur Gruppe der Pektinasen gehören die Pektinesterase (PE), die mazerierende oder nicht-mazerierende Polygalacturonase (PG) und die Pektin-Transeliminase (PTE), die auch als Pektinlyase bezeichnet wird. Bevorzugt wird das Verfahren der Erfindung zur Erzeugung der PE oder der - vorzugsweise mazerierenden - PG angewandt. Unter den technisch verfügbaren Schimmelpilzen der Art Aspergillus niger sind hervorragende Pektinasebildner bekannt, so daß sich deren Genom für das Verfahren der Erfindung eignet. Eine Steigerung ihrer Produktivität läßt sich durch das Verfahren der Erfindung erreichen, insbesondere wenn das Pektinase-Gen mehrfach in das Genom des Wirtsorganismus inseriert wird.

Als Wirtsorganismus eignet sich außer Aspergillus niger auch Aspergillus awamori. Der erfindungsgemäß genmodifizierte Schimmelpilz kann in an sich bekannter Weise durch Anlegen einer Kultur und Aufarbeitung des Kulturfiltrats zur Produktion von Pektinasen eingesetzt werden.

### Bevorzugte Ausführung der Erfindung

### Beispiel 1: Expression eines Polygalakturonase-Gens

Der Stamm Aspergillus niger DSM 5575 als Gen-Donor wurde in einem 30 l-Fermenter mit 4 % Weizenkleie, 4 % feingemahlenen Rübenschnitzeln und 0,2 % Ammoniumsulfat für 96 Stunden bei 28 Grad C, 1 VVM Belüftung und 600 Upm Rührergeschwindigkeit kultiviert.

Um auf der Grundlage der Proteinsequenz einer während der Fermentation gebildeten Polygalakturonase (PG) mit einem Molekulargewicht von ca. 38 000 Dalton und einem isoelektrischen Punkt von ca. 5,5 eine Oligonukleotid-Sonde zur Auffindung des Gens in einer cDNA-Bank von DSM 5575 ableiten zu können, wurde dieses Enzym aus dem Kulturüberstand gereinigt. Während der Reinigung wurde das Enzym anhand seiner Aktivität in Polygalakturonase (PG)-Einheiten gemäß der Technischen Information der Röhm GmbH Nr. FR-5-01-D vom 12.02.1987 sowie anhand der UV-Absorption bei 280 nm detektiert. Eine Polygalacturonase-Einheit ist definiert durch die Enzymaktivität, welche die Viskosität einer Standard-Pektinlösung um l/η ₛₚ = 0,000015 unter den Standardbedingungen der Analysenvorschrift senkt.

Dazu wurde der Fermentationsüberstand über ® Sephadex G25 Coarse (Pharmacia) entsalzt. Über Kationenaustauschchromatographie an ® Fractogel TSK CM-650 (Merck) bei pH 4,0 in 10 mM Natriumacetat/Acetat-Puffer, wurde die PG in einem linearen NaCl-Gradienten bei ca. 0,3 M eluiert.

Darauf folgt eine Gelfiltration über ® Sephacryl S 200 (Pharmacia), eine erneute Kationenaustauschchromatographie unter denselben Bedingungen wie oben genannt, jedoch mit ® Pharmacia Mono S als Gelmaterial. Die Endreinigung erfolgte durch nochmalige Gelfiltration über ® Sephacryl S 200 und ® Superose 12 (Pharmacia).

Ausgehend von dem gereinigten Enzym wurden nach Spaltungen mit Bromcyan, Trypsin und Staphylococcus V8 Protease Peptide präpariert und deren Aminosäuresequenzen unter Verwendung eines Gasphasensequenators (Applied Biosystems Model 470A) bestimmt. Dabei wurde die Online Detektion der Peptide mit einer 120 A HPLC vorgenommen. Die Bromcyanspaltungen erfolgten nach Groß, E. (1967), Methods in Enzymology 11, 238 - 255, die Peptidtrennung wurde auf einer HPLC Reverse Phase C4-Säule in 0,1 % Trifluoressigsäure/Propanol/ Acetonitril-Gradienten durchgeführt. Tryptische Spaltungen und Spaltungen mit der Staphylococcus V8 Protease wurden in 20 mM Ammoniumbicarbonat bei pH 7,8 ausgeführt. Die entsprechenden Peptide wurden wiederum durch Reverse Phase HPLC-Chromatographie, jedoch mit einer C18-Säule getrennt. Die N-terminale Sequenz des nativen Proteins wurde direkt bestimmt. Sie lautet:

Gly-Ser-Cys-Thr-Phe-Lys-Thr-Ala-Ala-Ala-

Ala-Lys-Ala-Gly-Lys-Ala-Gly-Cys-Ser-Thr-

Ile-Thr-Leu-Asp-Asn-Ile-Glu-Val-Pro-Ala-

Eines der nach Bromcyan-Spaltung erhaltenen Peptide, CN7, wurde als Vorlage für die Synthese des Oligonukleotids ER13 verwendet. Die Aminosäure-Sequenz Ile-Gln-Gln-Asp-Tyr-Glu im Peptid CN7 war wegen des gering degenerierten genetischen Codes dieser Aminosäuren für die Ableitung eines Oligonukleotids geeignet. Zur Festlegung der Nukleotidsequenz im Oligonukleotid ER13 wurden die am häufigsten verwendeten Codons im Glucoamylase-Gen aus A.niger, Boel, E. et al. (1984), EMBO J. 3, 1097-1102, und im Acetamidase-Gen aus A.nidulans, Corrick, C.M. et al. (1987), Gene 53, 63 - 71, zugrundegelegt. Da in beiden Genen keine eindeutige Codonpräferenz für Asparaginsäure ermittelt werden konnte, wurden die beiden möglichen Codons in ER13 eingesetzt.

Aminosäuresequenz von CN7:

Ser-Gly-Ile-Ser-Asp-Tyr-Gly-Val-Val-
Ile-Gln-Gln-Asp-Tyr-Glu-Asp-Gly-Lys

Ausgewählte Aminosäure-Sequenz für die Synthese von ER13: Ile-Gln-Gln-Asp-Tyr-Glu mögliche Codons:

      U
5'-AUC-CAA-CAA-GAU-UAU-GAA-3'
         G   G   C   G
      A

Sequenz des Oligonukleotids ER13 komplementär zur RNA:

                  A
3'- TAG-GTC-GTC-CT -ATG-CT-5'
                  G

Zur Präparation von mRNA wurde der Stamm DSM 5575 in Schüttelkolben unter PG-induzierenden Bedingungen, mit 2 % Weizenkleie, 2 % feingemahlenen Rübenschnitzeln und 0,1 % Ammoniumsulfat als Nährmedium für 96 Stunden auf einem Reziprokschüttler bei 28 Grad C kultiviert. Das erhaltene Mycel wurde abfiltriert und unmittelbar in flüssigem Stickstoff tiefgefroren.

Die Präparation von RNA erfolgte nach der Guanidiniumisothiocyanat/CsCl-Methode nach Maniatis, E. et al. (1982), Cold Spring Harbor Laboratory Press, New York, Seite 196. Aus der so gewonnenen Gesamt-RNA wurde durch Säulenchromatographie an Oligo(dT)-Cellulose Poly(A)⁺-mRNA angereichert, Taylor, J.M. (1979), Ann. Rev. Biochem. 48, 681 - 717.

Aus der mRNA wurde nach Gubler, U. und Hoffmann, B.J. (1983), Gene 25, 263 - 269, cDNA synthetisiert. Hierbei wurden ca. 5 ug mRNA mit 60 U M-MLV Reverser Transkriptase (GIBCO-BRL GmbH, 7514 Eggenstein, BRD) in einzelsträngige cDNA umgeschrieben. Aus 400 ng der einzelsträngigen cDNA konnten unter Verwendung von 0,4 U Ribonuklease H (GIBCO-BRD GmbH, 7514 Eggenstein, BRD) 10 U E. coli-DNA-Polymerase I (Boehringer Mannheim GmbH, 6800 Mannheim, BRD) und 0,9 U E. coli-DNA-Ligase (New England Biolabs GmbH Schwalbach, BRD) ca. 450 ng doppelsträngige cDNA gewonnen werden. Pufferzusammensetzungen und Nukleotidkonzentrationen entsprachen der Vorschrift von Gubler, U. und Hofmann, B.J. (1983), Gene 25, 263 - 269.

Zur Herstellung von glatten Enden wurden die cDNA-Enden mit Hilfe von 5 U T4-Polymerase (GIBCO-BRL GmbH, 7514 Eggenstein, BRD) und 50 µM Desoxynukleosidtriphosphaten nach Gubler, U. (1987), Methods in Enzymology Vol. 152, Seite 330 - 335, aufgefüllt. Zur Anreicherung von cDNA-Molekülen über 900 bp Länge wurde die cDNA über eine Biogel A50m-Säule nach Größe getrennt. Fraktionen, die cDNA-Moleküle über 1 kbp Länge enthielten wurden gesammelt. Zur Insertion der cDNA in dG-geteilten pUC9-Vektor (Pharmacia LKB GmbH, 7800 Freiburg, BRD) wurden 20 ng doppelsträngige cDNA mit über 1 kbp Länge mit einem 10⁴-fachen Überschuß an dCTP und 14,7 U terminaler Transferase (GIBCO-BRL GmbH, 7514 Eggenstein, BRD) für 7 min bei 37 Grad C in einem Gesamtvolumen von 40 µl inkubiert, Maniatis et al. (1982), Cold Spring Habor Laboratory Press, New York, Seite 241, und so mit homopolymeren dC-Resten versehen.

5 ng dieser cDNA wurden nach Gubler, U. und Hoffmann, B.J. (1983), Gene 25, 263-269, in 50 ng des pUC9-Vektors eingefügt. Die Transformation des rekombinanten Vektors erfolgte in kompetente E. coli DH5α ^{TM}-Zellen (GIBCO-BRL GmbH, 7514 Eggenstein, BRD) nach den Angaben des Herstellers. Auf diese Weise wurde eine ca. 5000 rekombinante Klone umfassende cDNA-Bank von Aspergillus niger DSM 5575 erhalten.

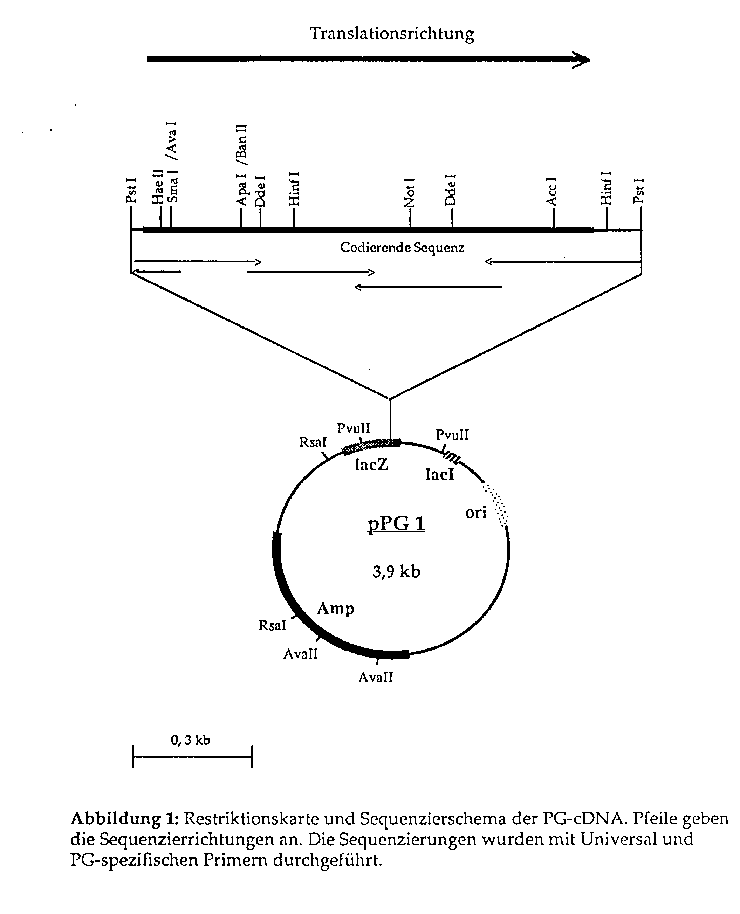

Die Aspergillus niger cDNA-Bank wurde mit dem (32)P-markierten Oligonukleotid ER13 gescreent. Dabei erfolgte die Hybridisierung bei 37 Grad C; gewaschen wurde jeweils 30 min bei 37 Grad C in Gegenwart von 6-fach SSC-Puffer, dann 2-fach SSC-Puffer, 2-fach SSC-Puffer mit 1 % SDS und schließlich 0,1-fach SSC-Puffer. Nach Autoradiographie der Nitrocellulosefilter konnten drei stark hybridisierende E.coli-Klone identifiziert werden, deren Plasmide nach Restriktionsanalysen und partiellen DNA-Sequenzierungen zeigten, daß alle 3 Insertionen identisch waren. In Abbildung 1 sind die Restriktionsanalyse und das Sequenzierschema der PG-cDNA einer Transformante dargestellt. Das rekombinante Plasmid wurde mit pPG1 bezeichnet. Die Sequenzierung der vollständigen DNA-Sequenz der PG-cDNA erfolgte nach Sanger, F. et al. (1977), Proc. Natl. Acad. Sci. USA 74, 5463-5467, mit Hilfe von Universal- und PG-spezifischen Sequenzierprimern. Die erhaltene cDNA-Sequenz ist in Tabelle 1 angegeben.

Die aus der cDNA abgeleitete Aminosäure-Sequenz zeigt weitestgehende Übereinstimmung mit den aus der Proteinsequenzierung ermittelten Peptidsequenzen.

Um einen Phagen-Klon mit den zur Expression in Aspergillus notwendigen flankierenden Bereichen aus der chromosomalen DNA von DSM 5575 zu erhalten, wurde zunächst chromosomale DNA nach einer Vorschrift von Hynes, M.J. et al. (1983), Mol. Cell. Biol. 3, 1430 - 1439, isoliert. Hierzu wurden etwa 10 g Mycel unter flüssigem Stickstoff zerrieben und nach Aufnahme in 10 mM Tris-HCl, pH 7,5,1 mM EDTA und 3 % SDS mit Phenol extrahiert. Die weitere Reinigung der DNA erfolgte durch Behandlung mit Proteinase K und RNase A. Die erhaltene hochmolekulare DNA wurde partiell mit Sau3A hydrolysiert und durch Saccharose-Dichtegradientenzentrifugation nach ihrer Größe fraktioniert. DNA-Moleküle der Größe von 18 - 22 kb wurden in BamHI/EcoRI-hydrolysierte EMBL3-DNA (Stratagene GmbH, 6900 Heidelberg, BRD) inseriert und in vitro verpackt. Die Verpackungsextrakte wurden ebenfalls von Stratagene GmbH bezogen. Nach Vermehrung der Phagen in E.coli P2392 (Stratagene GmbH) konnte eine ca. 70 000 rekombinante Klone umfassende Phagenbank angelegt werden.

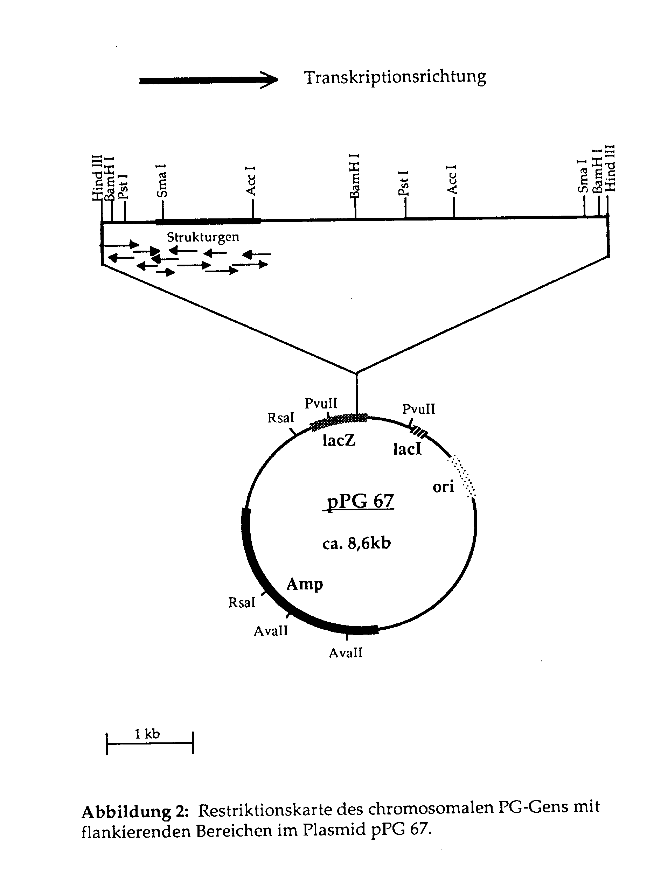

Die Phagen-DNA wurde nach Aufbringung auf Hybond-N-Membranen (Amersham Corp., Braunschweig) mit dem (32)P-markierten 1,3-kbp-PstI-Fragment aus pPG 1 gescreent. Die Hybridisierung erfolgte dabei bei 65 Grad C; gewaschen wurden die Filter jeweils 30 min bei 65 Grad C in 2-fach SSC-Puffer, dann 2-fach SSC-Puffer mit 1 % SDS und schließlich 0,1-fach SSC-Puffer. Aus ca. 70 000 rekombinanten EMBL3-Phagen wurden mehrere hybridisierende Klone erhalten. Aus einem davon wurde das einzige wiederum hybridisierende etwa 6 kb große HindIII-Fragment in pUC19 umkloniert und in E.coli DH5α transformiert. Das resultierende Plasmid, welches das vollständige chromosomale PG-Gen mit den zur Expression notwendigen flankierenden Sequenzen enthielt wurde als pPG67 bezeichnet. E.coli DH5α pPG67 wurde bei DSM unter der Nummer 5553 hinterlegt. Die Restriktionskarte von pPG67 ist in Abbildung 2 gezeigt. Die das Strukturgen sowie flankierende Bereiche aus pPG67 enthaltende DNA-Sequenz ist in Tabelle 2 wiedergegeben.

Die Stämme Aspergillus niger DSM 5575, A.awamori DSM 5574 und A.oryzae DSM 5573 wurden nach dem folgenden Protokoll transformiert.

100 ml Czapek-Dox-Komplett-Medium (Oxoid Czapek-Dox-Medium mit 0,1 % Hefeextrakt) in einem 1 Liter Erlenmeyer-Kolben mit Schikanen wurden mit ca. 10⁷ Sporen des jeweiligen Pilzstammes beimpft und 16 Stunden bei 37 Grad C auf einem Reziprokschüttler mit einer Frequenz von 120 Auslenkungen pro Minute inkubiert. Das Mycel wurde über ein Papierfilter geerntet und zweimal mit MP-Puffer (1,2 M MgSO4, 10 mM NaH₂PO₄, pH 5,8) gewaschen. Etwa 5 g des feuchten Mycels wurden in 15 ml MP-Puffer in einem 100 ml Erlenmeyer-Kölbchen ohne Schikanen aufgenommen. Der Suspension wurden 600 ul einer ® Novozym 234-Lösung (1 g in 6 ml MP-Puffer) und 100 µl β-Glucuronidase (Sigma) zugesetzt und der Ansatz für 5 Minuten in Eis gekühlt. Anschließend wurden 300 µl einer Rinderserumalbumin-Lösung (0,2 g in 4 ml MP-Puffer) zugegeben. Die Protoplastierung des Mycels erfolgt bei 30 Grad C in einem Wasserbadrundschüttler mit 12,5 mm Schütteldurchmesser bei 100 Umdrehungen pro Minute. Die Protoplastierungszeit betrug bei DSM 5575 und DSM 5574 ca. 3,5 bis 4 Stunden, bei DSM 5573 ca. 1,5 bis 2 Stunden. Der Protoplastierungszustand wurde mikroskopisch kontrolliert.

Die Protoplastensuspension wurde über ein mit MP-Puffer getränktes Glaswoll-Filter in Zentrifugenröhrchen gegeben und jeweils mit einem gleichen Volumen Ü-Puffer (600 mM Sorbitol, 100 mM Tris/HCl pH 7,0) überschichtet. Nach 10 minütiger Zentrifugation bei 20 Grad C und 2500 g wurden die Protoplasten aus der Schicht zwischen den beiden Pufferlösungen entnommen. Die erhaltene Suspension wurde nun mit zwei Volumina ST-Puffer (1,2 M Sorbitol, 10 mM Tris/HCl pH 7,5) gemischt und 10 Minuten bei 20 Grad C und 1500 g zentrifugiert. Das Protoplastensediment wurde anschließend in 10 ml STC-Puffer (1,2 M Sorbitol, 10 mM Tris/HCl pH 7,5, 10 mM CaCl₂) aufgenommen und wiederum bei 20 Grad C und 1500 g zentrifugiert. Der Vorgang wurde wiederholt und das Sediment anschließend in 1 ml STC-Puffer resuspendiert.

Da das Plasmid pPG67 keine Selektionsmarker für die zu transformierenden Aspergillus-Stämme besitzt wurde die Methode der Cotransformation angewandt. Für Aspergillus niger DSM 5575 und Aspergillus awamori DSM 5574 wurde dazu das Plasmid pAN7-1 (Punt et al., Gene 56, 117 -124), das ein in diesen Stämmen exprimierbares Hygromycin-Resistenz-Gen (Hygromycin-Phosphotransferase) enthält, verwendet. Pro Cotransformationsansatz wurden je 20 µg pAN7-1 und die gleiche Menge pPG67 eingesetzt. Da Aspergillus oryzae DSM 5573 eine natürliche Resistenz gegenüber Hygromycin B aufweist, wurde dieser Stamm mit dem Plasmid p3SR2 (Kelly und Hynes, EMBO Journal 4, 475 - 479) cotransformiert, das eine Selektion der Transformanten durch einen Wachstumsvorteil auf Minimalmedium mit Acetamid als alleiniger Stickstoffquelle durch die Expression eines Acetamidase-Gens ermöglicht. In den Cotransformationsansätzen wurden jeweils 40 µg p3SR2 und die gleiche Menge pPG67 eingesetzt.

Die zur Transformation eingesetzte DNA wurde in einem Volumen von 50 µl TE-Puffer (10 mM Tris/HCl pH 7,5, 1 mM EDTA) aufgenommen. Die Kontrollansätze erhielten jeweils 50 µl TE-Puffer ohne DNA, bzw. nur das Selektionsplasmid pAN7-1 oder p3SR2. 20 µl der DNA Lösung bzw. des TE-Puffers ohne DNA wurden zu 300 µl Protoplastensuspension gegeben und 10 Minuten bei 0 Grad C inkubiert. Nach nochmaliger Zugabe von 25 µl DNA-Lösung bzw. TE-Puffer ohne DNA und Zugabe von 400 µl PEG-Lösung (60 % Polyethylenglykol 4000 von Serva, 10 mM Tris/HCl pH 7,5, 50 mM CaCl2) erfolgte eine 5 minütige Inkubation bei 22 Grad C. Anschließend wurden 600 ul der PEG-Lösung zugegeben und der Ansatz für weitere 20 Minuten bei 22 Grad C inkubiert.

Im Falle der Hygromycin B-Selektion wurde der Ansatz in 100 ml Czapek-Dox-Agar (Oxoid mit 1 % Oxoid-Agar No. 1) mit 1 M Saccharose als osmotischem Stabilisator, der bei 45 Grad C flüssig gehalten wurde, gegeben und in jeweils 10 ml Portionen in Petrischalen verteilt. Nach 16 stündiger Inkubation bei 37 Grad C erfolgte die Hygromycin B (Calbiochem)-Zugabe in einer 10 ml Oberschicht des gleichen Agars jedoch ohne Saccharose in einer Konzentration von 200 µg/ml. Nach 2 - 4 Tagen weiterer Inkubation bei 37 Grad C konnten die Transformanten als aus der Agaroberfläche hervorbrechende versporende Kolonien, die sich deutlich von einem Hintergrundwachstum in der Agarschicht abhoben und in den Kontrollansätzen nicht vorhanden waren, identifiziert und isoliert werden. Die Transformanten wurden anschließend zweimal über Einzelsporpassagen auf Czapek-Dox-Agar mit 50 µg Hygromycin B/ml gereinigt. Die Ausgangsstämme DSM 5575 und DSM 5574 wachsen auf diesem Medium nicht. Die Transformationsraten lagen bei etwa 10 Transformanten pro µg pAN7-1 für DSM 5575 und bei etwa 20 Transformanten pro µg pAN7-1 für DSM 5574.

Im Falle der Acetamidase-Selektion wurde der Transformationsansatz mit STC-Puffer auf 10 ml aufgefüllt und 10 Minuten bei 20 Grad C und 1500 g zentrifugiert. Das Sediment wurde in 1 ml STC-Puffer resuspendiert und anschließend mit 9 ml Acetamid-Minimal-Agar mit 1 M Saccharose als osmotischem Stabilisator bei 45 Grad C gemischt. Der Agar wurde in 2 ml Portionen auf Acetamid-Minimal-Agar-Platten mit 1 M Saccharose und 15 mM CsCl zur Inhibition des Hintergrundwachstums verteilt. Nach 6 - 12 Tagen konnten die Transformanten als kräftig wachsende und versporende Kolonien, die sich deutlich vom Hintergrundwachstum abheben, identifiziert und isoliert werden. Die Transformanten wurden durch zweimalige Einzelsporpassagen auf Acetamid-Minimalagar ohne CsCl und Saccharose gereinigt. Die Zusammensetzung des Acetamid-Minimal-Nährbodens entsprach der von Kelly und Hynes (siehe oben). Die Transformationsrate für A.oryzae DSM 5573 lag bei etwa 2,5 Transformanten pro µg p3SR2.

Aus den Transformationsversuchen und den Kontrollansätzen wurden jeweils 50 Transformanten in Schüttelkolben auf ihre PG-Produktivität hin untersucht. Dabei wurden die gleichen Bedingungen wie bei der Mycelanzucht zur mRNA-Isolierung (siehe oben) gewählt. In Tabelle 3 sind die in den Kulturüberständen gefundenen PG-Aktivitäten in Einheiten/ml der jeweils besten Transformante angegeben. Da die Cotransformationsfrequenz, gemessen an der Zahl der Klone, die den Ausgangsstamm um mindestens das doppelte an PG-Produktivität übertrafen, in allen drei Versuchen > 30 % lag, wurde der beste Klon aus mindestens 15 Transformanten ausgewählt. Da in den Kontrollansätzen keine Transformante die Produktivität des Ausgangsstammes übertraf gilt der angegebene Wert für alle 50 Stämme. Wegen der in Schüttelkolben von Versuch zu Versuch gegebenen Schwankungen sind Wertebereiche angegeben.

Die besten Resultate wurden demnach in Aspergillus niger DSM 5575 (Transformante Nr. 7) mit 240 - 270 PG-Einheiten/ml erzielt. In Aspergillus awamori DSM 5574 (Transformante Nr. 12) wurde mit 120 - 150 PG-Einheiten/ml ein besseres Ergebnis als in Aspergillus oryzae DSM 5573 (Transformante Nr. 4) mit 20 - 30 PG-Einheiten/ml erhalten.

Die folgenden Tabellen erläutern die Erfindung näher. Es zeigen
**Tabelle 1** die Nucleotidsequenz der PG-cDNA aus Asp. niger und die daraus abgeleitete Aminosäuresequenz im 1-Buchstaben-Code. Start- und Stopcodon des offenen Leserahmens sind unterstrichen. Das N-terminale Ende (Aminosäuren 1 - 30) des nativen Proteins ist gekennzeichnet. Das Peptid CN7 ist durch Pfeile markiert. Die darin unterstrichenen Aminosäuren dienten zur Vorlage für die Synthese des Oligonukleotids ER13.
**Tabelle 2** die Nukleotidsequenz des chromosomalen PG-Gens aus dem Plasmid pPG67 und die daraus abgeleitete Aminosäuresequenz im 1-Buchstaben-Code. Die N-terminale Sequenz der nativen PG ist unterstrichen. Ferner sind das Intron 1 sowie die Start- und Stopcodons markiert.
**Tabelle 3** die in den Kulturüberständen gefundenen PG-Aktivitäten in Einheiten pro ml der jeweils besten Transformante.

Im 1-Buchstaben-Code werden folgende Codons verwendet:
A - Alanin, C - Cystein, D - Asparaginsäure, E - Glutaminsäure, F - Phenylalanin, G - Glycin, H - Histidin, I - Isoleucin, K - Lysin, L - Leucin, M - Methionin, N - Asparagin, P - Prolin, Q - Glutamin, R - Arginin, S - Serin, T - Threonin, V - Valin, W - Trypotophan, Y - Tyrosin.

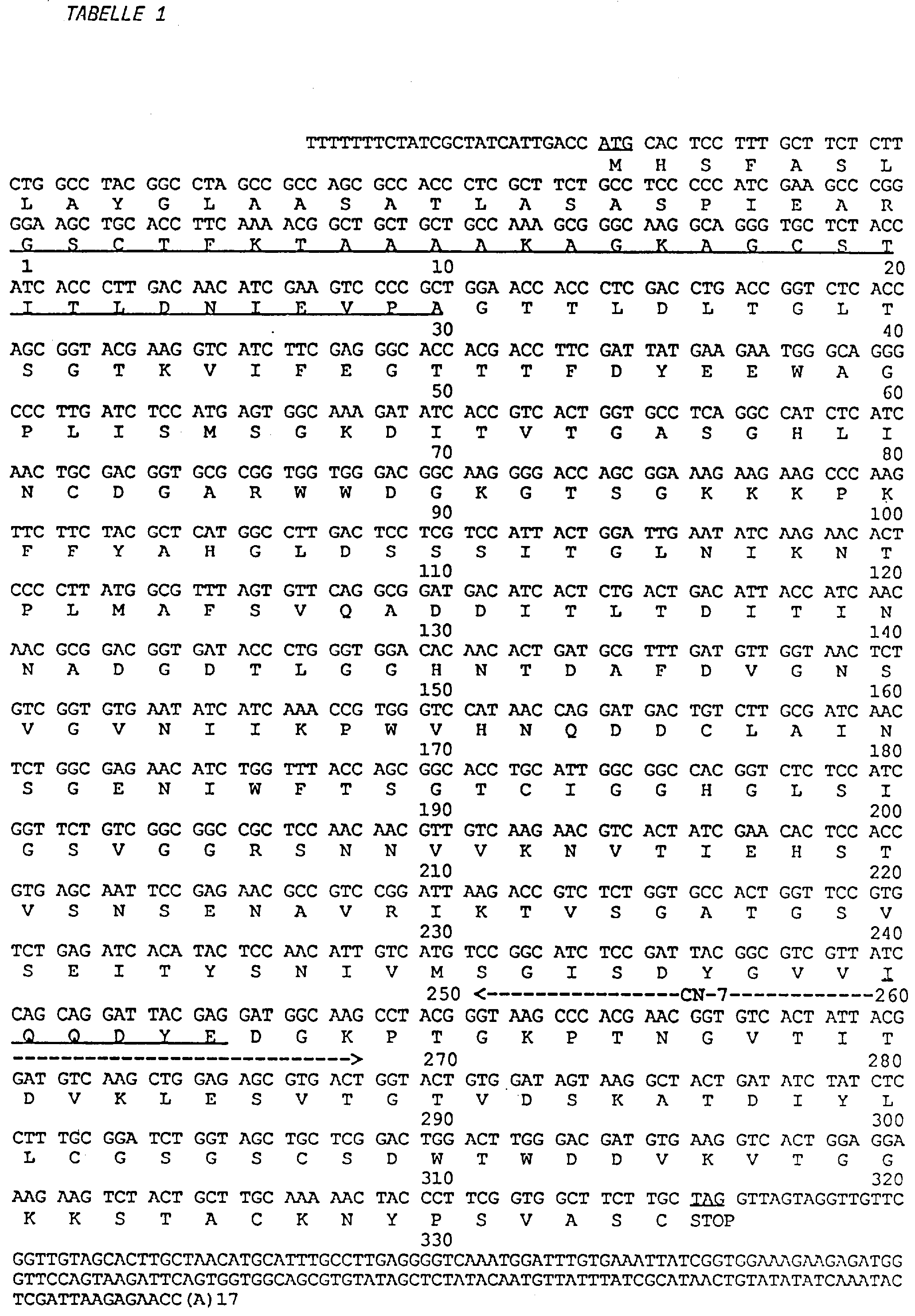

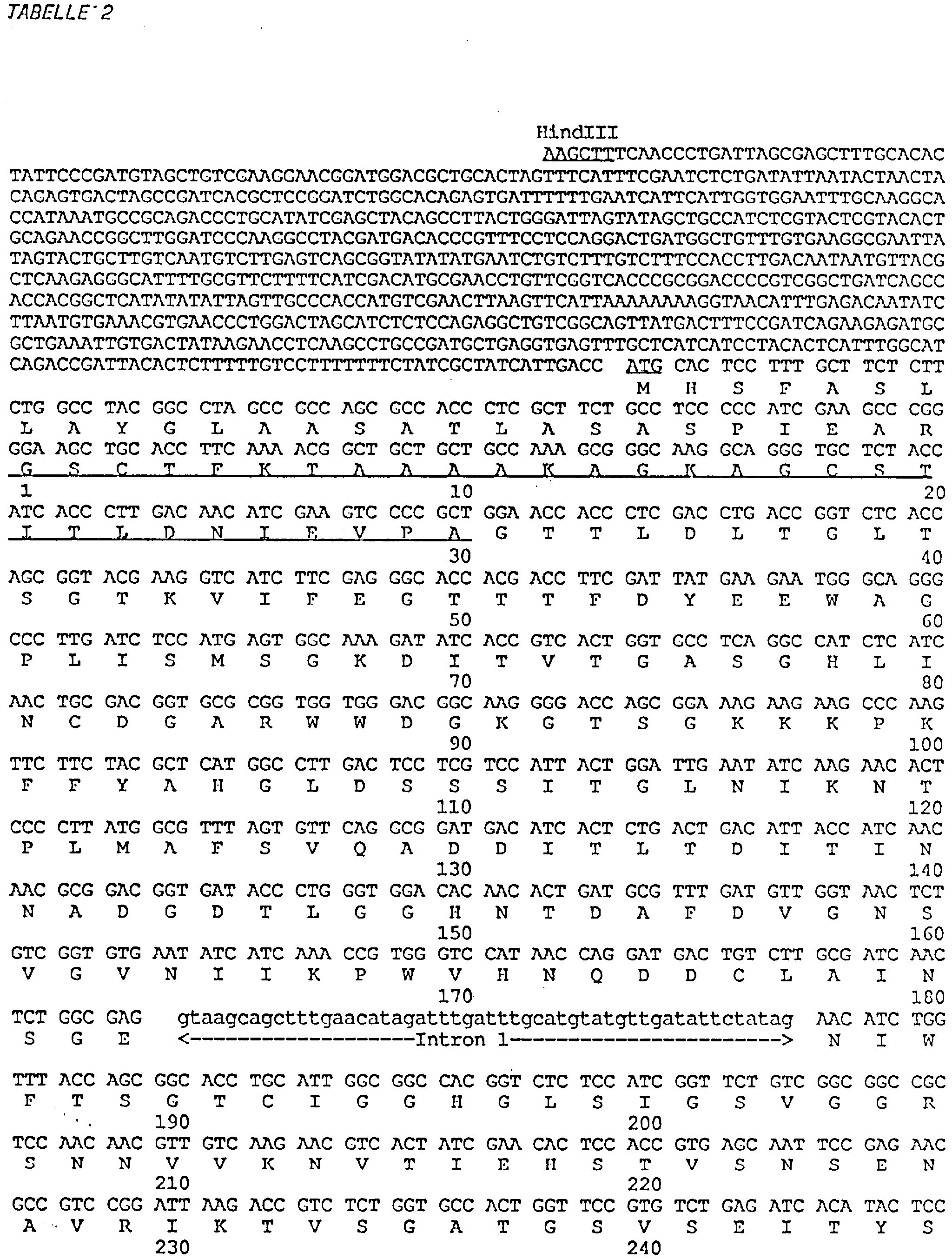

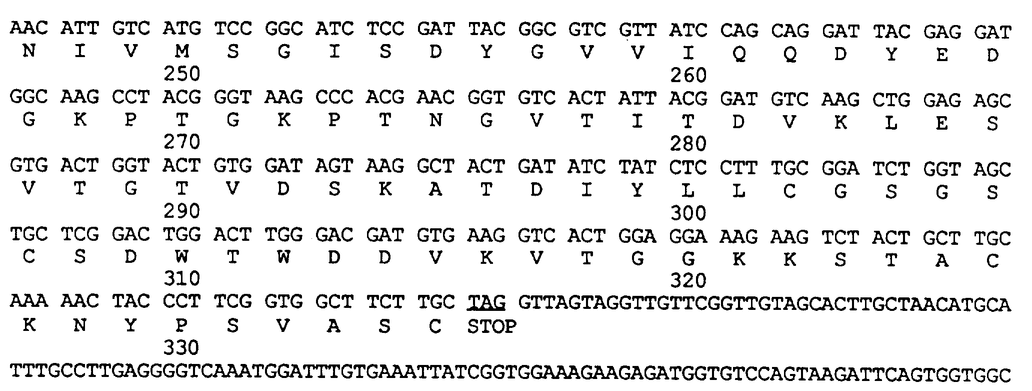

**TABELLE 3**

| | PG-Einheiten/ml |
|---|---|
| 1. Aspergillus niger | |
| DSM 5575 (Ausgangsstamm) | ca. 20 - 30 |
| DSM 5575 pAN7-1 (Transformanten 1-50) | ca. 20 - 30 |
| DSM 5575 pAN7-1/pPG67 (Transformante Nr. 7) | ca. 240 - 270 |

| 2. Aspergillus awamori | |
|---|---|
| DSM 5574 (Ausgangsstamm) | < 10 |
| DSM 5574 pAN7-1 (Transformanten 1-50) | < 10 |
| DSM 5574 pAN7-1/pPG67 (Transformante Nr. 12) | ca. 120 - 150 |

| 3. Aspergillus oryzae | |
|---|---|
| DSM 5573 (Ausgangsstamm) | < 10 |
| DSM 5573 p3SR2 (Transformante 1-50) | < 10 |
| DSM 5573 p3SR2/pPG67 (Transformante Nr. 4) | ca. 20 - 30 |

### Beispiel 2: Expression eines Pektinesterase (PE)-Gens

Der Stamm Aspergillus niger DSM 5575 wurde in einem 30 l MBR-Fermenter mit 4 % Weizenkleie, 4 % feingemahlenen Rübenschnitzeln und 0,2 % Ammoniumsulfat für 96 Stunden bei 28 Grad C, 1 VVM Belüftung und 600 Upm Rührergeschwindigkeit kultiviert.

Um auf der Grundlage der Proteinsequenz einer während der Fermentation gebildeten Pektinesterase (PE) mit einem Molekulargewicht von ca. 43 000 Dalton und einem isoelektrischen Punkt von ca. 3,6 eine Oligonukleotid-Sonde zur Auffindung des Gens in einer cDNA-Bank von DSM 5575 ableiten zu können, wurde dieses Enzym aus dem Kulturüberstand gereinigt. Während der Reinigung wurde das Enzym anhand seiner Aktivität in Pektinesterase (PE)-Einheiten gemäß der Technischen Information der Röhm GmbH Nr. FR-5-02-D vom 12.02.1987 sowie anhand der UV-Absorption bei 280 nm detektiert. Die Methode beruht auf der Titration der bei der Spaltung von Pektin freigesetzten COOH-Gruppen mit n/10 NaOH bei konstantem pH-Wert. Bei Verwendung einer 1-%igen Obi-Pektinlösung (reines Apfelpektin, Braunband, hochverestert, Fa. Obipektin AG, Bischofszell/Schweiz) wird die Abbaukurve bis zu einem Verbrauch von 2,5 ml n/10 NaOH gerade. Der pH-Wert bei der Verwendung von Obi-Pektin liegt bei 4,5. Die Aktivität wird in internationalen Einheiten angegeben.

Der Fermentationsüberstand wurde über ® Sephadex G25 Coarse (Pharmacia) entsalzt. Über Anionenaustauschchromatographie an ® Fractogel TSK DEAE-650 (Merck) bei pH 8,0 in 10 mM Tris/HCl-Puffer, wurde die PE in einem linearen NaCl-Gradienten bei ca. 0,3 M eluiert. Darauf folgt eine Gelfiltration über ® Sephacryl S 200 HR (Pharmacia), eine erneute Anionenaustauscherchromatographie unter denselben Bedingungen wie oben genannt, jedoch mit ® Pharmacia Mono Q als Gelmaterial. Die Endreinigung erfolgte durch hydrophobe Chromatographie an Phenyl-Sepharose CL 4B (Pharmacia).

Ausgehend von dem gereinigten Enzym wurden nach Spaltungen mit Bromcyan, Trypsin und Staphylococcus V8 Protease Peptide präpariert und deren Aminosäuresequenzen unter Verwendung eines Gasphasensequenators (Applied Biosystems Model 470A) bestimmt. Dabei wurde die Online Detektion der Peptide mit einer 120 A HPLC vorgenommen. Die Bromcyanspaltungen erfolgten nach Groß, E. (1967), Methods in Enzymology 11, 238 - 255, die Peptidtrennung wurde auf einer HPLC Reverse Phase C4-Säule in 0,1 % Trifluoressigsäure/Propanol/ Acetonitril-Gradienten durchgeführt. Tryptische Spaltungen und Spaltungen mit der Staphylococcus V8 Protease wurden in 20 mM Ammoniumbicarbonat bei pH 7,8 ausgeführt. Die entsprechenden Peptide wurden wiederum durch Reverse Phase HPLC-Chromatographie, jedoch mit einer C18-Säule getrennt. Die N-terminale Sequenz des nativen Proteins wurde direkt bestimmt. Sie lautet:

Ala-Ser-Arg-Met-Thr-Ala-Pro-Ser-Gly-Ala-

Ile-Val-Val-Ala-Lys-Ser-Gly-Gly-Asp-Tyr-

Asp-Thr-Ile-Ser-Ala-Ala-

Eines der nach trypischer Spaltung erhaltenen Peptide, T2-21, mit folgender N-terminaler Aminosäure-Sequenz wurde als Vorlage für die Synthese des Oligonukleotids PE 3 verwendet:

T2-21: Thr-Ser-Met-Thr-Asp-Val-Ile-Asn-His-Leu-Gly-
Trp-Thr-Glu-Trp

Die Synthese der komplementären Nukleotidsequenz der Gensonde PE 3 erfolgte nach dem von Beaucage, S.L. und Caruthers, M.H. (1981), Tetrahedron Letters 22, 1859 - 1862, entwickelten Phosphoramiditverfahren und wurde mittels eines DNA-Synthesizers (Applied Biosystems 380 A DNA Synthesizer, California, USA) durchgeführt. Die Codon-Auswahl wurde nach den bekannten Codon-Präferenzen des Clucoamylase-Gens aus A.niger (Boel, E. et al. (1984), EMBO J. 3, 1097 - 1102) getroffen. Die Nukleotidsequenz wurde vom Aminosäurebereich Met bis Trp abgeleitet. Entsprechend lautet die komplementäre Nukleotidsequenz der Gensonde PE 3.

PE 3:    3′-TAC TGG CTG CAG TAG TTG GTG GAG CCG ACC TGG
         CTC ACC - 5′

Zur Präparation von mRNA wurde der Stamm DSM 5575 in Schüttelkolben unter PE-induzierenden Bedingungen, mit 2 % Weizenkleie, 2 % feingemahlenen Rübenschnitzeln und 0,1 % Ammoniumsulfat als Nährmedium für 96 Stunden auf einem Reziprokschüttler bei 28 Grad C kultiviert. Das erhaltene Mycel wurde abfiltriert und unmittelbar in flüssigem Stickstoff tiefgefroren.

Die RNA-Präparation wurde nach der Methode von Chirgwin et al. (1979), Biochemistry 18, 5294 - 5299, durchgeführt. Die Isolierung von Poly(A)⁺-RNA aus Gesamt-RNA erfolgte durch eine Oligo(dT)-Celluloseaffinitätschromatographie (Avis, H. und Leder, P. (1972), Proc. Natl. Acad. Sci. USA, 69, 1408 - 1412): Die cDNA-Synthese wurde nach der Methode von Gubler, U. und Hoffmann, B.J. (1983) Gene 25, 263 - 269, modifiziert. Für die Synthese des ersten Stranges wurden in einem Reaktionsvolumen von 50 µl 10 µg Poly(A)⁺-RNA, 50 mM Tris-HCl, pH 8,3 5 mM MgCl₂, 75 mM KCl, 10 mM DTT, 1,0 mM dNTP (je 1,0 mM dATP, dCTP,dGTP,dTTP), 10 µg Oligo (dT₁₂₋₁₈) und 2000 U M-MLV Reverse Transkriptase (Gibco-BRL GmbH, 7514 Eggenstein, BRD) eingesetzt. Es wurde für 60 min bei 37 Grad C inkubiert. Anschließend wurde phenolisiert und eine Ethanolfällung durchgeführt.

Für die Zweitstrangsynthese wurde das RNA/DNA-Hybrid in einem Reaktionsvolumen von 100 µl mit folgenden Komponenten versetzt: 2 U RNase H (Gibco-BRL GmbH, 7514 Eggenstein, BRD), 1 U E.coli DNA-Ligase (New England Biolabs GmbH, 6231 Schwalbach, BRD), 25 U DNA-Polymerase I (Boehringer Mannheim GmbH, 6800 Mannheim, BRD), 20 mM Tris-HCl, pH 7,5, 100 mM KCl, 5 mM MgCl₂, 25 uM NAD, 100 mM (NH₄)₂SO₄, 10 mM DTT; 5 µg BSA und 60 µM dNTP (je 60 µM dATP, dCTP, dGTP, dTTP). Der Ansatz wurde 1 Stunde bei 14 Grad C und anschließend 1 Stunde bei 20 Grad C inkubiert.

Die cDNA-Fragmente einer Größe von 0,6 bis 1,7 kb wurden durch eine Biogel A50 (100 - 200 mesh)- Säurenchromatographie isoliert, in das mit SmaI linearisierte und dephosphorylierte Plasmid pUC 18 inseriert und anschließend in kompetente E. coli DH5α -Zellen (Hanahan, D. (1985) in: Glover D.M. (ed.) DNA cloning: A practical approach Vol. I, IRL Press Oxford, England, Seit 109 - 135) transformiert. Auf diese Weise wurde eine ca. 10 000 Klone umfassende cDNA-Bank von A. niger DSM 5575 erhalten.

Die cDNA-Klone wurden auf Nitrocellulosefilter übertragen und mit der (32)P-markierten Gensonde PE 3 bei 50 Grad C für 18 Stunden hybridisiert. Im Anschluß an die Hybridisierung wurden die Filter jeweils 30 min bei 68 Grad C mit 2-fach SSC-Puffer, 2-fach SSC-Puffer mit 0,1 % SDS und mit 0,2-fach SSC-Puffer gewaschen. Anschließend wurde für 48 Stunden bei -70 Grad C eine Autoradiographie durchgeführt.

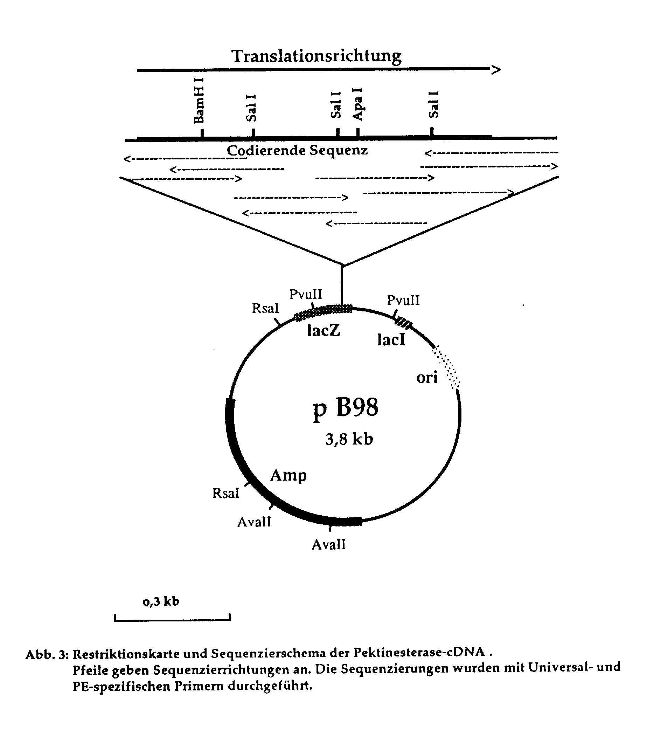

Insgesamt wurden 38 stark hybridisierende E. coli-Klone identifiziert, deren Plasmide zur weiteren Charakterisierung mittels Schnellpräparationsmethode (Birnboim, H.C. und Doly, J. (1979), Nucleic Acids Res. 7, 1513-1523) isoliert wurden. Die Plasmid-DNAs wurden mit EcoRI und HindIII hydrolysiert, die Fragmente elektrophoretisch getrennt, und anschließend wurde eine Southern-Hybridisierung mit der Gensonde PE 3 durchgeführt. Aufgrund der Southern-Blot-Analysen wurden 4 Klone mit einem cDNA-Insert zwischen 0,6 und 1,2 kb ausgewählt und doppelsträngig sequenziert. Die Sequenzierung wurde nach der Methode von Sanger, F. et al. (1977), Proc. Natl. Acad. Sci. USA, 74, 5463-5467, unter Verwendung von Universal- und PE-spezifischen Sequenzprimern durchgeführt. Vom längsten der 4 Klone sind die vollständige Nukleotidsequenz und die daraus abgeleitete Aminosäuresequenz in Tabelle 4 dargestellt. Das offene Leseraster umfaßt 993 Nukleotide und codiert für 331 Aminosäuren bzw. für 314 Aminosäuren ab der Übereinstimmung mit dem aus der Proteinsequenzierung erhaltenen N-Terminus. Das Plasmid erhielt die Bezeichnung pB98 (Abb. 3). Bis auf die 5 Aminosäuren in den Positionen 72, 275, 289, 290 und 292 stimmt die aus der cDNA abgeleitete Aminosäuresequenz mit den aus der Proteinsequenzierung ermittelten Peptidsequenzen überein. Die restlichen 3 Klone waren 600 bis 1100 Nukleotide lang und stimmten vollkommen mit der DNA-Sequenz des Klones pB98 überein.

Um einen Phagen-Klon mit den zur Expression in Aspergillus notwendigen flankierenden Bereichen aus der chromosomalen DNA von DSM 5575 zu erhalten, wurde zunächst chromosomale DNA nach einer Vorschrift von Hynes, M.J. et al. (1983), Mol. Cell. Biol. 3, 1430 - 1439, isoliert. Hierzu wurden etwa 10 g Mycel unter flüssigem Stickstoff zerrieben und nach Aufnahme in 10 mM Tris-HCl, pH 7,5, 1 mM EDTA und 3 % SDS mit Phenol extrahiert. Die weitere Reinigung der DNA erfolgte durch Behandlung mit Proteinase K und RNase A. Die erhaltene hochmolekulare DNA wurde partiell mit Sau3A hydrolysiert und durch Saccharose-Dichtegradientenzentrifugation nach ihrer Größe fraktioniert. DNA-Moleküle der Größe von 18 - 22 kb wurden im BamHI/EcoRI-hydrolysierte EMBL3-DNA (Stratagene GmbH, 6900 Heidelberg, BRD) inseriert und in vitro verpackt. Die Verpackungsextrakte wurden von Stratagene GmbH, 6900 Heidelberg, BRD, bezogen. Nach Vermehrung der Phagen in E. coli P2392 (Stratagene, 6900 Heidelberg, BRD) konnte eine ca. 70 000 rekombinante Klone umfassende Phagenbank angelegt werden.

Als radioaktive Probe für die Plaque-Hybridisierung wurde das 1,2-kbp-EcoRI/HindIII-Fragment des cDNA-Klones eingesetzt. Die Hybridisierung erfolgte bei 65 Grad C; gewaschen wurden die Filter jeweils 30 min bei 68 Grad C in 2-fach SSC-Puffer, dann 2-fach SSC-Puffer mit 0,1 % und schließlich in 0,1-fach SSC-Puffer. Nach der Hybridisierung und mehrfacher Vereinzelung ließen sich 10 positive Klone identifizieren.

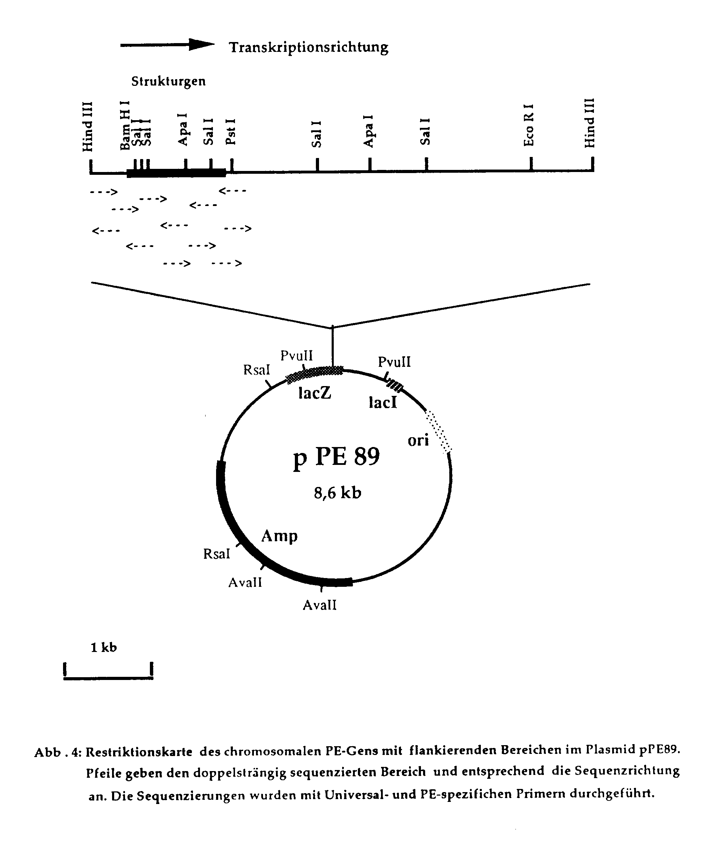

Die Phagen-DNA von Klon 35 wurde präpariert und mit HindIII hydrolysiert. Sie wies nach der Southern-Hybridisierung ein positives Signal bei ca. 6,0 kbp auf. Das HindIII-Fragment wurde mittels LMP-Agarose-Gelelektrophorese isoliert und in pUC 18 umkloniert und in E.coli DH5α transformiert. Das resultierende Plasmid, welches das vollständige chromosomale PE-Gen mit den zur Expression notwendigen flankierenden Sequenzen enthielt, wurde als pPE89 bezeichnet. Die Restriktionskarte von pPE89 ist in Abb. 4 dargestellt. Die das Strukturgen sowie flankierende Bereiche enthaltende DNA-Sequenz aus pPE89 wird in Tabelle 5 gezeigt. E.coli DH5 pPE89 wurde bei DSM unter der Nummer 5554 hinterlegt.

Transformationsprotokoll für Aspergillus niger DSM 5575, Aspergillus awamori DSM 5574 und Aspergillus oryzae DSM 5573.

100 ml Czapek-Dox-Komplett-Medium (Oxoid Czapek-Dox-Medium mit 0,1 % Hefeextrakt) in einem 1 Liter Erlenmeyer-Kolben mit Schikanen wurden mit ca. 10⁷ Sporen des jeweiligen Pilzstammes beimpft und 16 Stunden bei 37 Grad C auf einem Reziprokschüttler mit einer Frequenz von 120 Auslenkungen pro Minute inkubiert. Das Mycel wurde über ein Papierfilter geerntet und zweimal mit MP-Puffer (1,2 M MgSO4, 10 mM NaH₂PO₄, pH 5,8) gewaschen. Etwa 5 g des feuchten Mycels wurden in 15 ml MP-Puffer in einem 100 ml Erlenmeyer-Kölbchen ohne Schikanen aufgenommen. Der Suspension wurden 600 µl einer ^{R} Novozym 234-Lösung (1 g in 6 ml MP-Puffer) und 100 µl β-Glucuronidase (Sigma) zugesetzt und der Ansatz für 5 Minuten in Eis gekühlt. Anschließend wurden 300 µl einer Rinderserumalbumin-Lösung (0,2 g in 4 ml MP-Puffer) zugegeben. Die Protoplastierung des Mycels erfolgt bei 30 Grad C in einem Wasserbadrundschüttler mit 12,5 mm Schütteldurchmesser bei 100 Umdrehungen pro Minute. Die Protoplastierungszeit betrug bei DSM 5575 und DSM 5574 ca. 3,5 bis 4 Stunden, bei DSM 5573 ca. 1,5 bis 2 Stunden. Der Protoplastierungszustand wurde mikroskopisch kontrolliert.

Die Protoplastensuspension wurde über ein mit MP-Puffer getränktes Glaswoll-Filter in Zentrifugenröhrchen gegeben und jeweils mit einem gleichen Volumen Ü-Puffer (600 mM Sorbitol, 100 mM Tris/HCl pH 7,0) überschichtet. Nach 10 minütiger Zentrifugation bei 20 Grad C und 2500 g wurden die Protoplasten aus der Schicht zwischen den beiden Pufferlösungen entnommen. Die erhaltene Suspension wurde nun mit zwei Volumina ST-Puffer (1,2 M Sorbitol, 10 mM Tris/HCl pH 7,5) gemischt und 10 Minuten bei 20 Grad C und 1500 g zentrifugiert. Das Protoplastensediment wurde anschließend in 10 ml STC-Puffer (1,2 M Sorbitol, 10 mM Tris/HCl pH 7,5, 10 mM CaCl₂) aufgenommen und wiederum bei 20 Grad C und 1500 g zentrifugiert. Der Vorgang wurde wiederholt und das Sediment anschließend in 1 ml STC-Puffer resuspendiert.

Da das Plasmid pPE89 keine Selektionsmarker für die zu transformierenden Aspergillus-Stämme besitzt wurde die Methode der Cotransformation angewandt. Für Aspergillus niger DSM 5575 und Aspergillus awamori DSM 5574 wurde dazu das Plasmid pAN7-1 (Punt et al., Gene 56, 117 -124), das ein in diesen Stämmen exprimierbares Hygromycin-Resistenz-Gen (Hygromycin-Phosphotransferase) enthält, verwendet. Pro Cotransformationsansatz wurden je 20 µg pAN7-1 und die gleiche Menge pPE89 eingesetzt. Da Aspergillus oryzae DSM 5573 eine natürliche Resistenz gegenüber Hygromycin B aufweist, wurde dieser Stamm mit dem Plasmid p3SR2 (Kelly und Hynes, EMBO Journal 4, 475 - 479) cotransformiert, das eine Selektion der Transformanten durch einen Wachstumsvorteil auf Minimalmedium mit Acetamid als alleiniger Stickstoffquelle durch die Expression eines Acetamidase-Gens ermöglicht. In den Cotransformationsansätzen wurden jeweils 40 µg p3SR2 und die gleiche Menge pPE89 eingesetzt.

Die zur Transformation eingesetzte DNA wurde in einem Volumen von 50 µl TE-Puffer (10 mM Tris/HCl pH 7,5, 1 mM EDTA) aufgenommen. Die Kontrollansätze erhielten jeweils 50 µl TE-Puffer ohne DNA, bzw. nur das Selektionsplasmid pAN7-1 oder p3SR2. 20 µl der DNA Lösung bzw. des TE-Puffers ohne DNA wurden zu 300 µl Protoplastensuspension gegeben und 10 Minuten bei 0 Grad C inkubiert. Nach nochmaliger Zugabe von 25 µl DNA-Lösung bzw. TE-Puffer ohne DNA und Zugabe von 400 µl PEG-Lösung (60 % Polyethylenglykol 4000 von Serva, 10 mM Tris/HCl pH 7,5, 50 mM CaCl₂) erfolgte eine 5 minütige Inkubation bei 22 Grad C. Anschließend wurden 600 µl der PEG-Lösung zugegeben und der Ansatz für weitere 20 Minuten bei 22 Grad C inkubiert.

Im Falle der Hygromycin B-Selektion wurde der Ansatz in 100 ml Czapek-Dox-Agar (Oxoid mit 1 % Oxoid-Agar No. 1) mit 1 M Saccharose als osmotischem Stabilisator, der bei 45 Grad C flüssig gehalten wurde, gegeben und in jeweils 10 ml Portionen in Petrischalen verteilt. Nach 16 stündiger Inkubation bei 37 Grad C erfolgte die Hygromycin B (Calbiochem)-Zugabe in einer 10 ml Oberschicht des gleichen Agars jedoch ohne Saccharose in einer Konzentration von 200 µg/ml. Nach 2 - 4 Tagen weiterer Inkubation bei 37 Grad C konnten die Transformanten als aus der Agaroberfläche hervorbrechende versporende Kolonien, die sich deutlich von einem Hintergrundwachstum in der Agarschicht abhoben und in den Kontrollansätzen nicht vorhanden waren, identifiziert und isoliert werden. Die Transformanten wurden anschließend zweimal über Einzelsporpassagen auf Czapek-Dox-Agar mit 50 µg Hygromycin B/ml gereinigt. Die Ausgangsstämme DSM 5575 und DSM 5574 wachsen auf diesem Medium nicht. Die Transformationsraten lagen bei etwa 10 Transformanten pro µg pAN7-1 für DSM 5575 und bei etwa 20 Transformanten pro µg pAN7-1 für DSM 5574.

Im Falle der Acetamidase-Selektion wurde der Transformationsansatz mit STC-Puffer auf 10 ml aufgefüllt und 10 Minuten bei 20 Grad C und 1500 g zentrifugiert. Das Sediment wurde in 1 ml STC-Puffer resuspendiert und anschließend mit 9 ml Acetamid-Minimal-Agar mit 1 M Saccharose als osmotischem Stabilisator bei 45 Grad C gemischt. Der Agar wurde in 2 ml Portionen auf Acetamid-Minimal-Agar-Platten mit 1 M Saccharose und 15 mM CsCl zur Inhibition des Hintergrundwachstums verteilt. Nach 6 - 12 Tagen konnten die Transformanten als kräftig wachsende und versporende Kolonien, die sich deutlich vom Hintergrundwachstum abheben, identifiziert und isoliert werden. Die Transformanten wurden durch zweimalige Einzelsporpassagen auf Acetamid-Minimalagar ohne CsCl und Saccharose gereinigt. Die Zusammensetzung des Acetamid-Minimal-Nährbodens entsprach der von Kelly und Hynes (siehe oben). Die Transformationsrate für A.oryzae DSM 5573 lag bei etwa 2,5 Transformanten pro ug p3SR2.

Aus den Transformationsversuchen und den Kontrollansätzen wurden jeweils 50 Transformanten in Schüttelkolben auf ihre PE-Produktivität hin untersucht. Dabei wurden die gleichen Bedingungen wie bei der Mycelanzucht zur mRNA-Isolierung (siehe oben) gewählt. In Tabelle 6 sind die in den Kulturüberständen gefundenen PE-Aktivitäten in Einheiten/ml der jeweils besten Transformante angegeben. Da die Cotransformationsfrequenz, gemessen an der Zahl der Klone, die den Ausgangsstamm um mindestens das doppelte an PE-Produktivität übertrafen, in allen drei Versuchen > 30 % lag, wurde der beste Klon aus mindestens 15 Transformanten ausgewählt. Da in den Kontrollansätzen keine Transformante die Produktivität des Ausgangsstammes übertraf gilt der angegebene Wert für alle 50 Stämme. Wegen der in Schüttelkolben von Versuch zu Versuch gegebenen Schwankungen sind Wertebereiche angegeben.

Die besten Resultate wurden demnach in Aspergillus niger DSM 5575 (Transformante Nr. 9) mit 5,5 - 6,5 PE-Einheiten/ml erzielt. In Aspergillus awamori DSM 5574 (Transformante Nr. 17) wurde mit 2,5 - 3,0 PE-Einheiten/ml ein besseres Ergebnis als in Aspergillus oryzae DSM 5573 (Transformante Nr. 42) mit 0,8 - 1,0 PE-Einheiten/ml erhalten.

Die folgenden Tabellen erläutern die Erfindung näher. Es zeigen
**Tabelle 4** die Nucleotidsequenz der PE-cDNA aus Asp. niger und die daraus abgeleitete Aminosäuresequenz im 1-Buchstaben-Code. Start- und Stopcodon des offenen Leserahmens sowie das N-terminale Ende (Aminosäuren 1 - 26) des nativen Proteins und die PE3-Gensonde sind gekennzeichnet.
**Tabelle 5** die Nukleotidsequenz des chromosomalen PE-Gens aus dem Plasmid pPE89 und die daraus abgeleitete Aminosäuresequenz im 1-Buchstaben-Code. Die Introns 1 bis 6 sowie das Stopcodon sind gekennzeichnet.
**Tabelle 6** die in den Kulturüberständen gefundenen PE-Aktivitäten in Einheiten pro ml der jeweils besten Transformante.

Die Mikroorganismen DSM 5553 (E.coli DH5α pPG67), DSM 5554 (E.coli DH5α pPE89), DSM 5573 (Asp.oryzae RH3011), DSM 5574 (Asp.awamori RH 3630) und DSM 5575 (Asp.niger RH 5344) wurden am 29. September 1989 bei der DSM (Deutsche Sammlung von Mikroorganismen und Zellkulturen GmbH, Mascheroder Weg 1b, D-3300 Braunschweig) gemäß dem Budapester Vertrag hinterlegt.

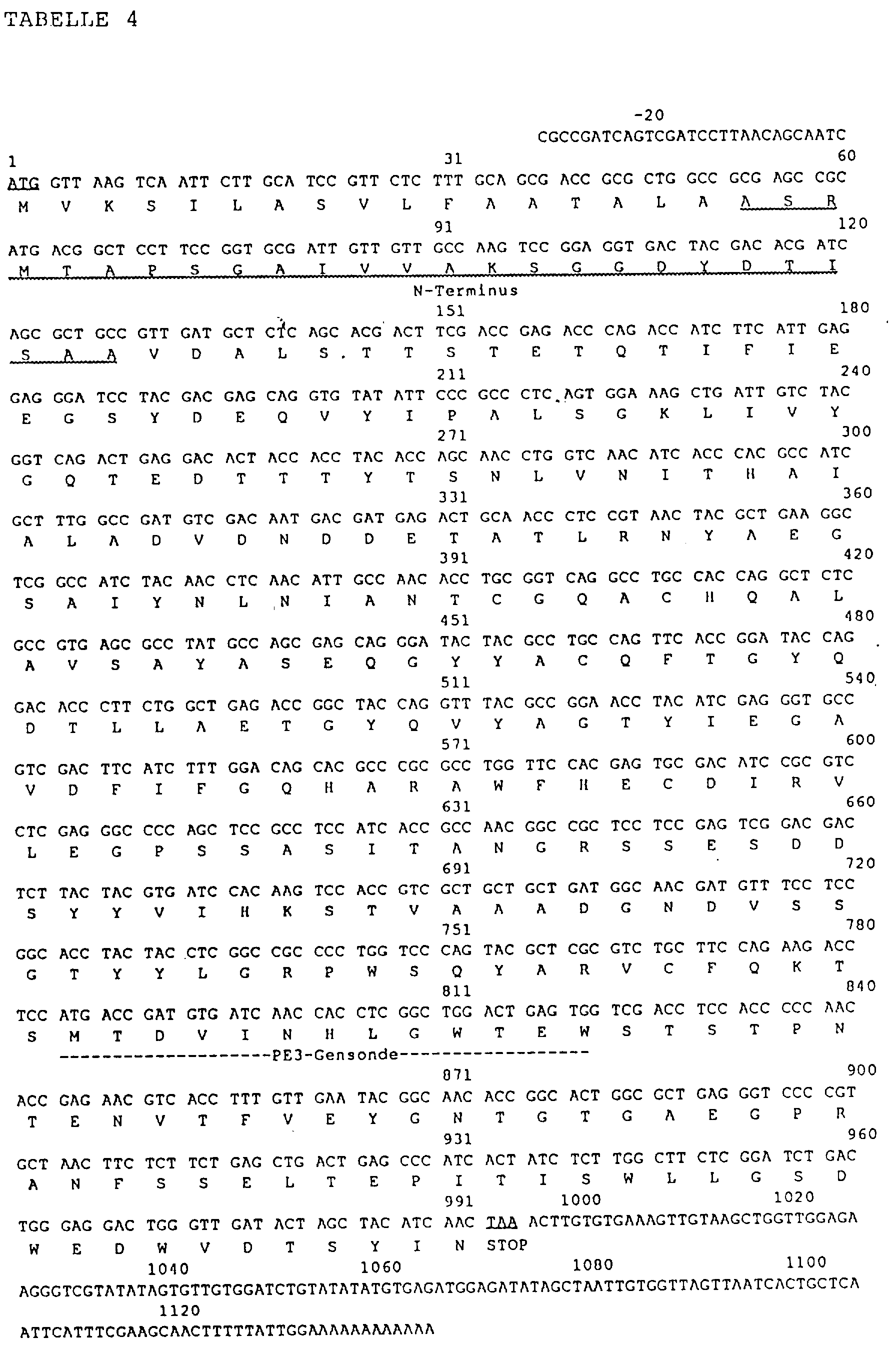

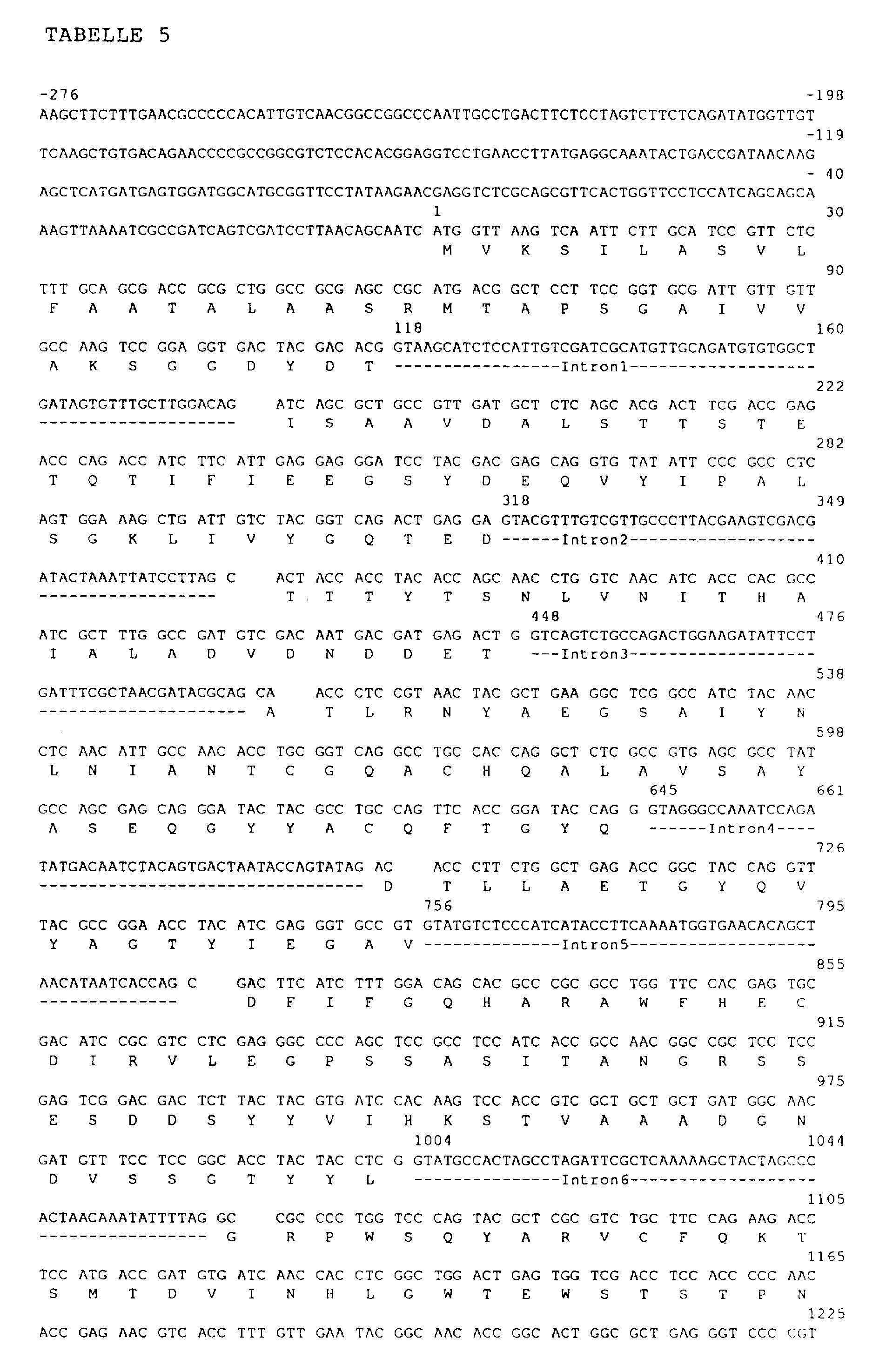

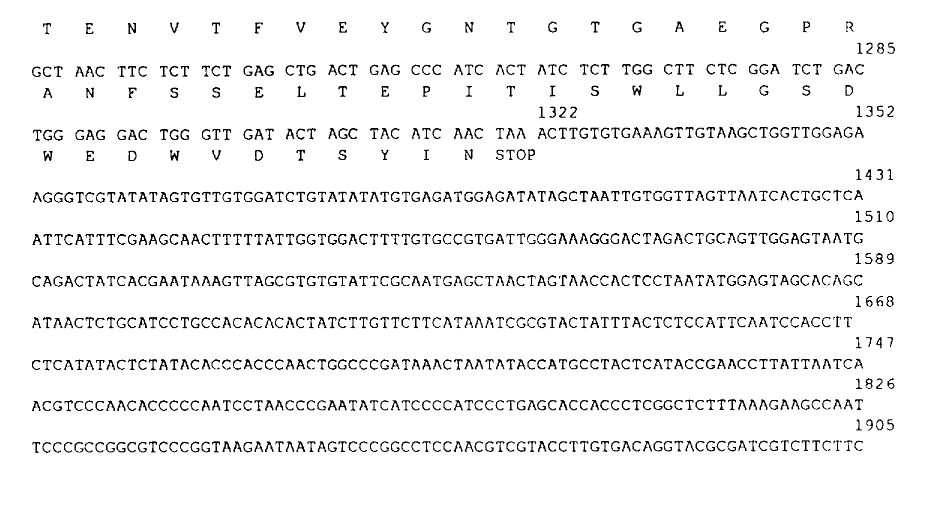

**TABELLE 6**

| | PE-Einheiten/ml |
|---|---|
| 1. Aspergillus niger | |
| DSM 5575 (Ausgangsstamm) | ca. 0,1 - 0,2 |
| DSM 5575 pAN7-1 (Transformanten 1-50) | ca. 0,1 - 0,2 |
| DSM 5575 pAN7-1/pPE89 (Transformante Nr. 9) | ca. 5,5 - 6,5 |

| 2. Aspergillus awamori | |
|---|---|
| DSM 5574 (Ausgangsstamm) | < 0,1 |
| DSM 5574 pAN7-1 (Transformanten 1-50) | < 0,1 |
| DSM 5574 pAN7-1/pPE89 (Transformante Nr. 17) | ca. 2,5 - 3,0 |

| 3. Aspergillus oryzae | |
|---|---|
| DSM 5573 (Ausgangsstamm) | < 0,1 |
| DSM 5573 p3SR2 (Transformante 1-50) | < 0,1 |
| DSM 5573 p3SR2/pPE89 (Transformante Nr. 42) | ca. 0,8 - 1,0 |

## Patentansprüche

1. Desoxyribonucleinsäure aus Aspergillus niger, deren Nucleotidsequenz Polygalakturonase oder Pektinesterase codiert, dadurch **gekennzeichnet**, daß die Polygalakturonase folgende Nucleotidsequenz 
GGA AGC TGC ACC TTC AAA ACG GCT GCT GCT GCC AAA GCG GGC AAG GCA GGG TGC TCT ACC
ATC ACC CTT GAC AAC ATC GAA GTC CCC GCT GGA ACC ACC CTC GAC CTG ACC GGT CTC ACC
AGC GGT ACG AAG GTC ATC TTC GAG GGC ACC ACG ACC TTC GAT TAT GAA GAA TGG GCA GGG
CCC TTG ATC TCC ATG AGT GGC AAA GAT ATC ACC GTC ACT GGT GCC TCA GGC CAT CTC ATC
AAC TGC GAC GGT GCG CGG TGG TGG GAC GGC AAG GGG ACC AGC GGA AAG AAG AAG CCC AAG
TTC TTC TAC GCT CAT GGC CTT GAC TCC TCG TCC ATT ACT GGA TTG AAT ATC AAG AAC ACT
CCC CTT ATG GCG TTT AGT GTT CAG GCG GAT GAC ATC ACT CTG ACT GAC ATT ACC ATC AAC
AAC GCG GAC GGT GAT ACC CTG GGT GGA CAC AAC ACT GAT GCG TTT GAT GTT GGT AAC TCT
GTC GGT GTG AAT ATC ATC AAA CCG TGG GTC CAT AAC CAG GAT GAC TGT CTT GCG ATC AAC
TCT GGC GAG AAC ATC TGG TTT ACC AGC GGC ACC TGC ATT GGC GGC CAC GGT CTC TCC ATC
GGT TCT GTC GGC GGC CGC TCC AAC AAC GTT GTC AAG AAC GTC ACT ATC GAA CAC TCC ACC
GTG AGC AAT TCC GAG AAC GCC GTC CGG ATT AAG ACC GTC TCT GGT GCC ACT GGT TCC GTG
TCT GAG ATC ACA TAC TCC AAC ATT GTC ATG TCC GGC ATC TCC GAT TAC GGC GTC GTT ATC
CAG CAG GAT TAC GAG GAT GGC AAG CCT ACG GGT AAG CCC ACG AAC GGT GTC ACT ATT ACG
GAT GTC AAG CTG GAG AGC GTG ACT GGT ACT GTG GAT AGT AAG GCT ACT GAT ATC TAT CTC
CTT TGC GGA TCT GGT AGC TGC TCG GAC TGG ACT TGG GAC GAT GTG AAG GTC ACT GGA GGA
AAG AAG TCT ACT GCT TGC AAA AAG TAC CCT TCG GTG GCT TCT TGC
 oder eine Nucleotidsequenz, die für die gleiche Aminosäuresequenz codiert, sich jedoch aufgrund der Degeneriertheit des genetischen Codes von der gezeigten Nukleotidsequenz unterscheidet, besitzt, und die Pektinesterase folgende Nucleotidsequenz 
GCG AGC CGC ATG ACG GCT CCT TCC GGT GCG ATT GTT GTT GCC AAG TCC GGA
GGT GAC TAC GAC ACG ATC AGC GCT GCC GTT GAT GCT CTC AGC ACG ACT TCG
ACC GAG ACC CAG ACC ATC TTC ATT GAG GAG GGA TCC TAC GAC GAG CAG GTG
TAT ATT CCC GCC CTC AGT GGA AAG CTG ATT GTC TAC GGT CAG ACT GAG GAC
ACT ACC ACC TAC ACC AGC AAC CTG GTC AAC ATC ACC CAC GCC ATC GCT TTG
GCC GAT GTC GAC AAT GAC GAT GAG ACT GCA ACC CTC CGT AAC TAC GCT GAA
GGC TCG GCC ATC TAC AAC CTC AAC ATT GCC AAC ACC TGC GGT CAG GCC TGC
CAC CAG GCT CTC GCC GTG AGC GCC TAT GCC AGC GAG CAG GGA TAC TAC GCC
TGC CAG TTC ACC GGA TAC CAG GAC ACC CTT CTG GCT GAG ACC GGC TAC CAG
GTT TAC GCC GGA ACC TAC ATC GAG GGT GCC GTC GAC TTC ATC TTT GGA CAG
CAC GCC CGC GCC TGG TTC CAC GAG TGC GAC ATC CGC GTC CTC GAG GGC CCC
AGC TCC GCC TCC ATC ACC GCC AAC GGC CGC TCC TCC GAG TCG GAC GAC TCT
TAC TAC GTG ATC CAC AAG TCC ACC GTC GCT GCT GCT GAT GGC AAC GAT GTT
TCC TCC GGC ACC TAC TAC CTC GGC CGC CCC TGG TCC CAG TAC GCT CGC GTC
TGC TTC CAG AAG ACC TCC ATG ACC GAT GTG ATC AAC CAC CTC GGC TGG ACT
GAG TGG TCG ACC TCC ACC CCC AAC ACC GAG AAC GTC ACC TTT GTT GAA TAC
GGC AAC ACC GGC ACT GGC GCT GAG GGT CCC CGT GCT AAC TTC TCT TCT GAG
CTG ACT GAG CCC ATC ACT ATC TCT TGG CTT CTC GGA TCT GAC TGG GAG GAC
TGG GTT GAT ACT AGC TAC ATC AAC
 oder eine Nucleotidsequenz, die für die gleiche Aminosäuresequenz codiert, sich jedoch aufgrund der Degeneriertheit des genetischen Codes von der gezeigten Nukleotidsequenz unterscheidet, besitzt.

2. Desoxyribonucleinsäure nach Anspruch 1, dadurch **gekennzeichnet,** daß sie aus Aspergillus niger DSM 5575 stammt.

3. Desoxyribonucleinsäure nach Anspruch 1 oder 2, dadurch **gekennzeichnet,** daß sie Polygalakturonase aus Aspergillus niger DSM 5575 codiert.

4. Desoxyribonucleinsäure nach Anspruch 1 oder 2, dadurch **gekennzeichnet,** daß sie Pektinesterase aus Aspergillus niger DSM 5575 codiert.

5. Verfahren zur Herstellung eines rekombinanten Wirtsorganismus, welcher mit einem aus Aspergillus stammenden Polygalakturonase-Gen oder Pektinesterase-Gen transformiert ist, durch Klonieren eines rekombinanten Vektors, der das Polygalakturonase-Gen oder Pektinesterase-Gen sowie die zur Expression in Aspergillus notwendigen flankierenden Desoxyribonucleotid-Sequenzen enthält und Transformation des rekombinanten Vektors in einen Wirtsorganismus, dadurch **gekennzeichnet,** daß als Polygalakturonase-Gen oder Pektinesterase -Gen eine Desoxyribonucleinsäure aus Aspergillus niger nach einem der Ansprüche 1 bis 4 oder eine entsprechende chromosomale Desoxyribonucleinsäure verwendet wird, und daß als Wirtsorganismus ein Schimmelpilz der Art Aspergillus niger oder Aspergillus awamori eingesetzt wird.

6. Verfahren nach Anspruch 5, dadurch **gekennzeichnet,** daß die folgenden Schritte durchgeführt werden:
(a) Kultivierung von Aspergillus niger unter Polygalakturonase- oder Pektinesterase-induzierenden Bedingungen,
(b) Reinigung der Polygalakturonase oder Pektinesterase und partielle Sequenzierung der Proteine,
(c) Isolierung der Messenger-Ribonucleinsäuren aus den unter (a) kultivierten Zellen,
(d) Erstellen einer Copy-Desoxyribonucleinsäurebank aus den Ribonucleinsäuren aus (c),
(e) Herstellung einer Oligodesoxyribonucleotidsonde, die aus der Proteinsequenz aus (b) abgeleitet wurde,
(f) Klonieren der das Polygalakturonase-Gen oder das Pektinesterase-Gen enthaltenden Copy-Desoxyribonucleinsäure unter Verwendung der Oligodesoxyribonucleotidsonde aus (e) als Hybridisierungsprobe,
(g) Erstellen einer Genbank, die die chromosomale Desoxyribonucleinsäure des unter (a) verwendeten Aspergillus-Stammes enthält,
(h) Klonieren eines rekombinanten Vektors aus der Genbank nach (g), welcher das chromosomale Polygalakturonase-Gen oder das Pektinesterase-Gen sowie zur Expression in Aspergillus notwendige flankierende Desoxyribonucleotid-sequenzen enthält, unter Verwendung der Copy-Desoxyribonucleinsäure aus (f) als Hybridisierungsprobe,
(i) Umklonieren des Polygalakturonase-Gens oder Pektinesterase-Gens sowie der zur Expression in Aspergillus notwendigen flankierenden Desoxyribonucleotid-Sequenzen in ein Plasmid,
(k) Cotransformation eines Aspergillus-niger- oder Aspergillus-awamori-Wirtsorganismus mittels des rekombinierten Vektors nach (i) und eines zweiten Plasmids, das einen in dem Wirtsorganismus exprimierbaren Selektionsmarker enthält,
(1) Selektion eines cotransformierten Stammes aus (k), dessen Polygalakturonase- oder Pektinesterase-Produktivität höher als die des unter (a) verwendeten Aspergillus niger ist.

7. Verfahren nach Anspruch 5 oder 6, dadurch **gekennzeichnet,** daß zur Herstellung des rekombinanten Wirtsorganismus eine chromosomale Desoxyribonucleinsäure, die Polygalakturonase aus Aspergillus niger codiert und folgende Nucleotidsequenz 
AAGCTTTCAACCCTGATTAGCGAGCTTTGCACACTATTCCCGATGTAGCTGTCGAAGGAACGGAT
GGACGCTGCACTAGTTTCATTTCGAATCTCTGATATTAATACTAACTACAGAGTGACTA
GCCGATCACGCTCCGGATCTGGCACAGAGTGATTTTTGAATCATTCATTGGTGGAATT
TGCAAGGCACCATAAATGCCGCAGACCCTGCATATCGAGCTACAGCCTTACTGGGATTA
GTATAGCTGCCATCTCGTACTCGTACACTGCAGAACCGGCTTGGATCCCAAGGCCTACG
ATGACACCCGTTTCCTCCAGGACTGATGGCTGTTTGTGAAGGCGAATTATAGTACTGCT
TGTCAATGTCTTGAGTCAGCGGTATATATGAATCTGTCTTTGTCTTTCCACCTTGACAA
TAATGTTACGCTCAAGAGGGCATTTTGCGTTCTTTTCATCGACATGCGAACCTGTTCGG
TCACCCGCGGACCCCGTCGGCTGATCAGCCACCACGGCTCATATATATTAGTTGCCCAC
CATGTCGAACTTAAGTTCATTAAAAAAAGGTAACATTTGAGACAATATCTTAATGTGA
AACGTGAACCCTGGACTAGCATCTCTCCAGAGGCTGTCGGCAGTTATGACTTTCCGATC
AGAAGAGATGCGCTGAAATTGTGACTATAAGAACCTCAAGCCTGCCGATGCTGAGGTGA
GTTTGCTCATCATCCTACACTCATTTGGCATCAGACCGATTACACTCTTTTTGTCCTTT
TTTTCTATCGCTATCATTGACC ATG CAC TCC TTT GCT TCT CTT CTG GCC
TAC GGC CTA GCC GCC AGC GCC ACC CTC GCT TCT GCC TCC CCC ATC
GAA GCC CGG GGA AGC TGC ACC TTC AAA ACG GCT GCT GCT GCC AAA
GCG GGC AAG GCA GGG TGC TCT ACC ATC ACC CTT GAC AAC ATC GAA
GTC CCC GCT GGA ACC ACC CTC GAC CTG ACC GGT CTC ACC AGC GGT
ACG AAG GTC ATC TTC GAG GGC ACC ACG ACC TTC GAT TAT GAA GAA
TGG GCA GGG CCC TTG ATC TCC ATG AGT GGC AAA GAT ATC ACC GTC
ACT GGT GCC TCA GGC CAT CTC ATC AAC TGC GAC GGT GCG CGG TGG
TGG GAC GGC AAG GGG ACC AGC GGA AAG AAG AAG CCC AAG TTC TTC
TAC GCT CAT GGC CTT GAC TCC TCG TCC ATT ACT GGA TTG AAT ATC
AAG AAC ACT CCC CTT ATG GCG TTT AGT GTT CAG GCG GAT GAC ATC
ACT CTG ACT GAC ATT ACC ATC AAC AAC GCG GAC GGT GAT ACC CTG
GGT GGA CAC AAC ACT GAT GCG TTT GAT GTT GGT AAC TCT GTC GGT
GTG AAT ATC ATC AAA CCG TGG GTC CAT AAC CAG GAT GAC TGT CTT
GCG ATC AAC TCT GGC GAG gtaagcagctttgaacatagatttgatttgcatg
<---------------INTRON 1---------
tatgttgatattctatag AAC ATC TGG TTT ACC AGC GGC ACC TGC ATT
------------------>
GGC GGC CAC GGT CTC TCC ATC GGT TCT GTC GGC GGC CGC TCC AAC
AAC GTT GTC AAG AAC GTC ACT ATC GAA CAC TCC ACC GTG AGC AAT
TCC GAG AAC GCC GTC CGG ATT AAG ACC GTC TCT GGT GCC ACT GGT
TCC GTG TCT GAG ATC ACA TAC TCC AAC ATT GTC ATG TCC GGC ATC
TCC GAT TAC GGC GTC GTT ATC CAG CAG GAT TAC GAG GAT GGC AAG
CCT ACG GGT AAG CCC ACG AAC GGT GTC ACT ATT ACG GAT GTC AAG
CTG GAG AGC GTG ACT GGT ACT GTG GAT AGT AAG GCT ACT GAT ATC
TAT CTC CTT TGC GGA TCT GGT AGC TGC TCG GAC TGG ACT TGG GAC
GAT GTG AAG GTC ACT GGA GGA AAG AAG TCT ACT GCT TGC AAA AAC
TAC CCT TCG GTG GCT TCT TGC TAG GTTAGTAGGTTGTTCGGTTGTAGCAC
TTGCTAACATGCATTTGCCTTGAGGGGTCAAATGGATTTGTGAAATTATCGGTGGAAAG
AAGAGATGGTGTCCAGTAAGATTCAGTGGTGGC
 oder eine Nucleotidsequenz, die für die gleiche Aminosäuresequenz codiert, sich jedoch aufgrund der Degeneriertheit des genetischen Codes von der gezeigten Nukleotidsequenz unterscheidet, besitzt, verwendet wird.

8. Verfahren nach Anspruch 5 oder 6, dadurch **gekennzeichnet,** daß zur Herstellung des rekombinanten Wirtsorganismus eine chromosomale Desoxyribonucleinsäure, die Pektinesterase aus Aspergillus niger codiert und folgende Nucleotidsequenz 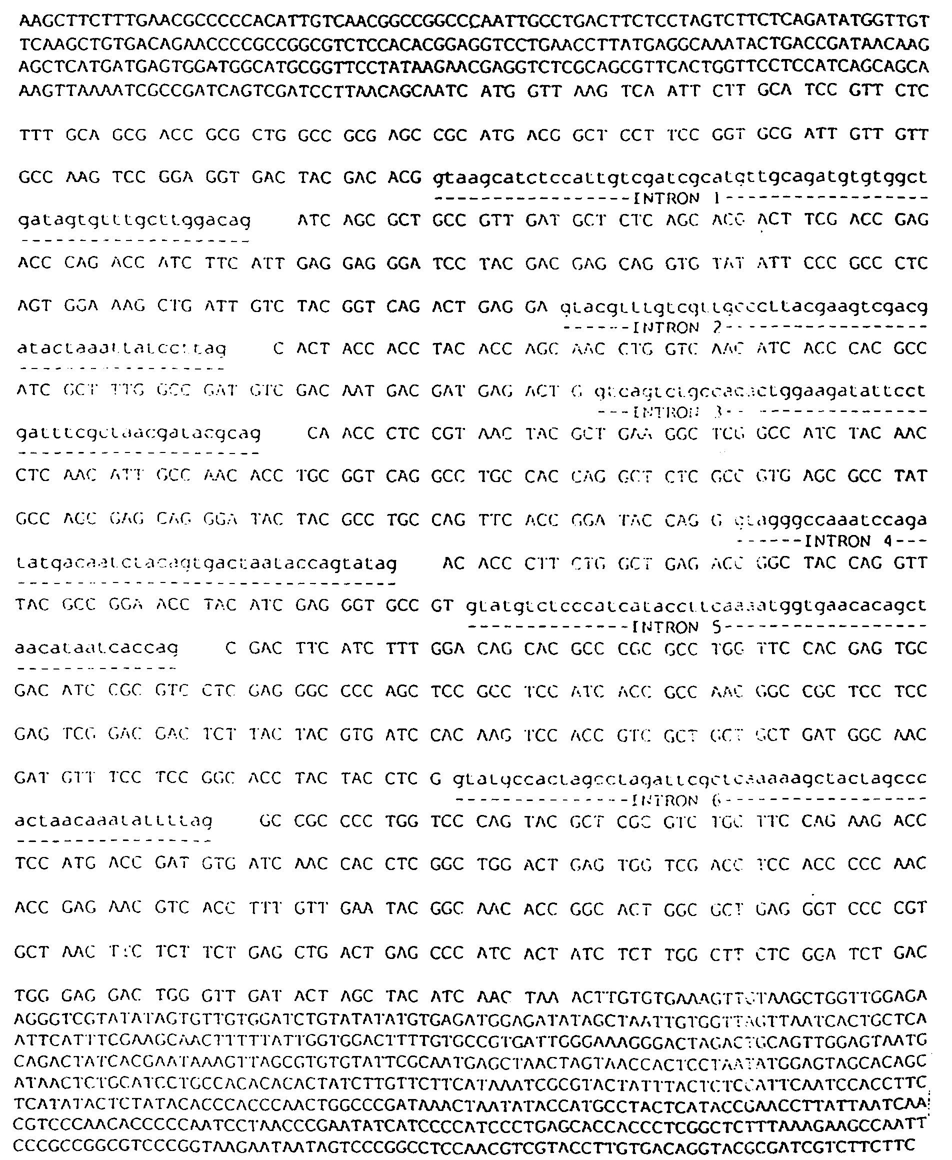 oder eine Nucleotidsequenz, die für die gleiche Aminosäuresequenz codiert, sich jedoch aufgrund der Degeneriertheit des genetischen Codes von der gezeigten Nukleotidsequenz unterscheidet, besitzt, verwendet wird.

9. Verfahren nach mindestens einem der Ansprüche 5 bis 8, dadurch **gekennzeichnet**, daß die Desoxyribonucleinsäure aus Aspergillus niger DSM 5575 stammt.

10. Verfahren nach Anspruch 9, dadurch **gekennzeichnet**, daß die Desoxyribonucleinsäure Polygalakturonase aus Aspergillus niger DSM 5575 codiert.

11. Verfahren nach Anspruch 9, dadurch **gekennzeichnet**, daß die Desoxyribonucleinsäure Pektinesterase aus Aspergillus niger DSM 5575 codiert.

12. Verfahren nach mindestens einem der Ansprüche 5 bis 11, dadurch **gekennzeichnet**, daß als Wirtsorganismus die Schimmelpilzstämme Aspergillus niger DSM 5575 oder Aspergillus awamori 5574 eingesetzt werden.

13. Verfahren nach mindestens einem der Ansprüche 5 bis 12, dadurch **gekennzeichnet**, daß das Pektinase-Gen mehrfach in das Genorn des Wirtsorganismus inseriert wird.

14. Verfahren nach mindestens einem der Ansprüche 5 bis 13, dadurch **gekennzeichnet**, daß eine Desoxyribonucleinsäure eingesetzt wird, die eine mazerierende Polygalakturonase codiert.

15. Verfahren zur Herstellung von Polygalakturonase oder Pektinesterase, dadurch **gekeinzeichnet,** daß der nach mindestens einem der Ansprüche 5 bis 14 erhaltene rekombinante Wirtsorganismus in einem geeigneten Nährmedium vermehrt wird und die Polygalakturonase oder Pektinesterase in an sich bekannter Weise isoliert wird.

## Claims

1. Deoxyribonucleic acid from Aspergillus niger, the nucleotide sequence of which encodes polygalacturonase or pectinesterase, characterised in that the polygalacturonase comprises the following nucleotide sequence 
GGA AGC TGC ACC TTC AAA ACG GCT GCT GCT GCC AAA GCG GGC AAG GCA GGG TGC TCT ACC
ATC ACC CTT GAC AAC ATC GAA GTC CCC GCT GGA ACC ACC CTC GAC CTG ACC GGT CTC ACC
AGC GGT ACG AAG GTC ATC TTC GAG GGC ACC ACG ACC TTC GAT TAT GAA GAA TGG GCA GGG
CCC TTG ATC TCC ATG AGT GGC AAA GAT ATC ACC GTC ACT GGT GCC TCA GGC CAT CTC ATC
AAC TGC GAC GGT GCG CGG TGG TGG GAC GGC AAG GGG ACC AGC GGA AAG AAG AAG CCC AAG
TTC TTC TAC GCT CAT GGC CTT GAC TCC TCG TCC ATT ACT GGA TTG AAT ATC AAG AAC ACT
CCC CTT ATG GCG TTT AGT GTT CAG GCG GAT GAC ATC ACT CTG ACT GAC ATT ACC ATC AAC
AAC GCG GAC GGT GAT ACC CTG GGT GGA CAC AAC ACT GAT GCG TTT GAT GTT GGT AAC TCT
GTC GGT GTG AAT ATC ATC AAA CCG TGG GTC CAT AAC CAG GAT GAC TGT CTT GCG ATC AAC
TCT GGC GAG AAC ATC TGG TTT ACC AGC GGC ACC TGC ATT GGC GGC CAC GGT CTC TCC ATC
GGT TCT GTC GGC GGC CGC TCC AAC AAC GTT GTC AAG AAC GTC ACT ATC GAA CAC TCC ACC
GTG AGC AAT TCC GAG AAC GCC GTC CGG ATT AAG ACC GTC TCT GGT GCC ACT GGT TCC GTG
TCT GAG ATC ACA TAC TCC AAC ATT GTC ATG TCC GGC ATC TCC GAT TAC GGC GTC GTT ATC
CAG CAG GAT TAC GAG GAT GGC AAG CCT ACG GGT AAG CCC ACG AAC GGT GTC ACT ATT ACG
GAT GTC AAG CTG GAG AGC GTG ACT GGT ACT GTG GAT AGT AAG GCT ACT GAT ATC TAT CTC
CTT TGC GGA TCT GGT AGC TGC TCG GAC TGG ACT TGG GAC GAT GTG AAG GTC ACT GGA GGA
AAG AAG TCT ACT GCT TGC AAA AAG TAC CCT TCG GTG GCT TCT TGC
 or a nucleotide sequence which encodes for the same amino acid sequence but which differs from the indicated nucleotide sequence due to the degeneracy of the genetic code, and which encodes pectinesterase of the following nucleotide sequence 
GCG AGC CGC ATG ACG GCT CCT TCC GGT GCG ATT GTT GTT GCC AAG TCC GGA
GGT GAC TAC GAC ACG ATC AGC GCT GCC GTT GAT GCT CTC AGC ACG ACT TCG
ACC GAG ACC CAG ACC ATC TTC ATT GAG GAG GGA TCC TAC GAC GAG CAG GTG
TAT ATT CCC GCC CTC AGT GGA AAG CTG ATT GTC TAC GGT CAG ACT GAG GAC
ACT ACC ACC TAC ACC AGC AAC CTG GTC AAC ATC ACC CAC GCC ATC GCT TTG
GCC GAT GTC GAC AAT GAC GAT GAG ACT GCA ACC CTC CGT AAC TAC GCT GAA
GGC TCG GCG ATC TAC AAC CTC AAC ATT GCC AAC ACC TGC GGT CAG GCC TGC
CAC CAG GCT CTC GCC GTG AGC GCC TAT GCC AGC GAG CAG GGA TAC TAC GCC
TGC CAG TTC ACC GGA TAC CAG GAC ACC CTT CTG GCT GAG ACC GGC TAC CAG
GTT TAC GCC GGA ACC TAC ATC GAG GGT GCC GTC GAC TTC ATC TTT GGA CAG
CAC GCC CGC GCC TGG TTC CAC GAG TGC GAC ATC CGC GTC CTC GAG GGC CCC
AGC TCC GCC TCC ATC ACC GCC AAC GGC CGC TCC TCC GAG TCG GAC GAC TCT
TAC TAC GTG ATC CAC AAG TCC ACC GTC GCT GCT GCT GAT GGC AAC GAT GTT
TCC TCC GGC ACC TAC TAC CTC GGC CGC CCC TGG TCC CAG TAC GCT CGC GTC
TGC TTC CAG AAG ACC TCC ATG ACC GAT GTG ATC AAC CAC CTC GGC TGG ACT
GAG TGG TCG ACC TCC ACC CCC AAC ACC GAG AAC GTC ACC TTT GTT GAA TAC
GGC AAC ACC GGC ACT GGC GCT GAG GGT CCC CGT GCT AAC TTC TCT TCT GAG
CTG ACT GAG CCC ATC ACT ATC TCT TGG CTT CTC GGA TCT GAC TGG GAG GAC
TGG GTT GAT ACT AGC TAC ATC AAC
 or a nucleotide sequence which encodes for the same amino acid sequence but which differs from the indicated nucleotide sequence due to the degeneracy of the genetic code.

2. Deoxyribonucleic acid according to claim 1, characterised in that it originates from Aspergillus niger DSM 5575.

3. Deoxyribonucleic acid according to claim 1 or 2, characterised in that it encodes polygalacturonase from Aspergillus niger DSM 5575.

4. Deoxyribonucleic acid according to claim 1 or 2, characterised in that it encodes pectinesterase from Aspergillus niger DSM 5575.

5. A method of producing a recombinant host organism, which is transformed with a polygalacturonase gene or pectinesterase gene originating from Aspergillus, by cloning a recombinant vector which contains the polygalacturonase gene or pectinesterase gene and contains the flanking deoxyribonucleotide sequences necessary for expression in Aspergillus, and transformation of the recombinant vector into a host organism, characterised in that a deoxyribonucleic acid from Aspergillus niger according to any one of claims 1 to 4 or a corresponding chromosomal deoxyribonucleic acid is used as the polygalacturonase gene or pectinesterase gene, and that a **fungus of the Asperillus niger or Asperillus awamori type is used as the host organism.**

6. A method according to claim 5, characterised in that the following steps are carried out:
(a) cultivation of Aspergillus niger under polygalacturonase-inducing or pectinesterase-inducing conditions,
(b) purification of the polygalacturonase or pectinesterase and partial sequencing of the proteins,
(c) isolation of the messenger ribonucleic acids from the cells cultivated in (a),
(d) preparation of a copy deoxyribonucleic acid bank from the ribonucleic acids from (c),
(e) preparation of an oligodeoxyribonucleic acid **probe** which has been derived from the protein sequence from (b),
(f) cloning the copy deoxyribonucleic acid containing the polygalacturonase gene or the pectinesterase gene using the oligodeoxyribonucleic acid **probe** from (e) as a hybridisation specimen,
(g) preparation of a gene bank which contains the chromosomal deoxyribonucleic acid of the Aspergillus strain used in (a),
(h) cloning a recombinant vector from the gene bank according to (g), which contains the chromosomal polygalacturonase gene or the pectinesterase gene and which contains the flanking deoxyribonucleotide sequences necessary for expression in Aspergillus, using the copy deoxyribonucleic acid from (f) as the hybridisation specimen,
(i) transcloning the polygalacturonase gene or pectinesterase gene and the flanking deoxyribonucleotide sequences necessary for expression in Aspergillus into a plasmid,
(k) cotransformation of an Aspergillus niger or Aspergillus awamori host organism by means of the recombinant vector according to (i) and a second plasmid which contains a selection marker which can be expressed in the host organism,
(I) selecting a cotransformed strain from (k), the polygalacturonase or pectinesterase productivity of which is higher than the Aspergillus niger used in (a).

7. A method according to claim 5 or 6, characterised in that a chromosomal deoxyribonucleic acid which encodes polygalacturonase from Aspergillus niger and which comprises the following nucleotide sequence 
AAGCTTTCAACCCTGATTAGCGAGCTTTGCACACTATTCCCGATGTAGCTGTCGAAGGAACGGAT
GGACGCTGCACTAGTTTCATTTCGAATCTCTGATATTAATACTAACTACAGAGTGACTA
GCCGATCACGCTCCGGATCTGGCACAGAGTGATTTTTGAATCATTCATTGGTGGAATT
TGCAAGGCACCATAAATGCCGCAGACCCTGCATATCGAGCTACAGCCTTACTGGATTA
GTATAGCTGCCATCTCGTACTCGTACACTGCAGAACCGGCTTGGATCCCAAGGCCTACG
ATGACACCCGTTTCCTCCAGGACTGATGGCTGTTTGTGAAGGCGAATTATAGTACTGCT
TGTCAATGTCTTGAGTCAGCGGTATATATGAATCTGTCTTTGTCTTTCCACCTTGACAA
TAATGTTACGCTCAAGAGGGCATTTTGCGTTCTTTTCATCGACATGCGAACCTGTTCGG
TCACCCGCGGACCCCGTCGGCTGATCAGCCACCACGGCTCATATATATTAGTTGCCCAC
CATGTCGAACTTAAGTTCATTAAAAAAAGGTAACATTTGAGACAATATCTTAATGTGA
AACGTGAACCCTGGACTAGCATCTCTCCAGAGGCTGTCGGCAGTTATGACTTTCCGATC
AGAAGAGATGCGCTGAAATTGTGACTATAAGAACCTCAAGCCTGCCGATGCTGAGGTGA
GTTTGCTCATCATCCTACACTCATTTGGCATCAGACCGATTACACTCTTTTTGTCCTTT
TTTTCTATCGCTATCATTGACC ATG CAC TCC TTT GCT TCT CTT CTG GCC
TAC GGC CTA GCC GCC AGC GCC ACC CTC GCT TCT GCC TCC CCC ATC
GAA GCC CGG GGA AGC TGC ACC TTC AAA ACG GCT GCT GCT GCC AAA
GCG GGC AAG GCA GGG TGC TCT ACC ATC ACC CTT GAC AAC ATC GAA
GTC CCC GCT GGA ACC ACC CTC GAC CTG ACC GGT CTC ACC AGC GGT
ACG AAG GTC ATC TTC GAG GGC ACC ACG ACC TTC GAT TAT GAA GAA
TGG GCA GGG CCC TTG ATC TCC ATG AGT GGC AAA GAT ATC ACC GTC
ACT GGT GCC TCA GGC CAT CTC ATC AAC TGC GAC GGT GCG CGG TGG
TGG GAC GGC AAG GGG ACC AGC GGA AAG AAG AAG CCC AAG TTC TTC
TAC GCT CAT GGC CTT GAC TCC TCG TCC ATT ACT GGA TTG AAT ATC
AAG AAC ACT CCC CTT ATG GCG TTT AGT GTT CAG GCG GAT GAC ATC
ACT CTG ACT GAC ATT ACC ATC AAC AAC GCG GAC GGT GAT ACC CTG
GGT GGA CAC AAC ACT GAT GCG TTT GAT GTT GGT AAC TCT GTC GGT
GTG AAT ATC ATC AAA CCG TGG GTC CAT AAC CAG GAT GAC TGT CTT
GCG ATC AAC TCT GGC GAG gtaagcagctttgaacatagatttgatttgcatg
<----------------INTRON 1---------
tatgttgatattctatag AAC ATC TGG TTT ACC AGC GGC ACC TGC ATT
------------------>
GGC GGC CAC GGT CTC TCC ATC GGT TCT GTC GGC GGC CGC TCC AAC
AAC GTT GTC AAG AAC GTC ACT ATC GAA CAC TCC ACC GTG AGC AAT
TCC GAG AAC GCC GTC CGG ATT AAG ACC GTC TCT GGT GCC ACT GGT
TCC GTG TCT GAG ATC ACA TAC TCC AAC ATT GTC ATG TCC GGC ATC
TCC GAT TAC GGC GTC GTT ATC CAG CAG GAT TAC GAG GAT GGC AAG
CCT ACG GGT AAG CCC ACG AAC GGT GTC ACT ATT ACG GAT GTC AAG
CTG GAG AGC GTG ACT GGT ACT GTG GAT AGT AAG GCT ACT GAT ATC
TAT CTC CTT TGC GGA TCT GGT AGC TGC TCG GAC TGG ACT TGG GAC
GAT GTG AAG GTC ACT GGA GGA AAG AAG TCT ACT GCT TGC AAA AAC
TAC CCT TCG GTG GCT TCT TGC TAG GTTAGTAGGTTGTTCGGTTGTAGCAC
TTGCTAACATGCATTTGCCTTGAGGGGTCAAATGGATTTGTGAAATTATCGGTGGAAAG
AAGAGATGGTGTCCAGTAAGATTCAGTGGTGGC
 or a nucleotide sequence which encodes for the same amino acid sequence but which differs from the indicated nucleotide sequence due to the degeneracy of the genetic code, is used for the production of the recombinant host organism.

8. A method according to claim 5 or 6, characterised in that a chromosomal deoxyribonucleic acid which encodes pectinesterase from Aspergillus niger and which comprises the following nucleotide sequence 
AAGCTTCTTTGAACGCCCCCACATTGTCAACGGCCGGCCCAATTGCCTGACTTCTCCTAGTCTTCTCAGATATGGTTGT
TCAAGCTGTGACAGAACCCCGCCGGCGTCTCCACACGGAGGTCCTGAACCTTATGAGGCAAATACTGACCGATAACAAG
AGCTCATGATGAGTGGATGGCATGCGGTTCCTATAAGAACGAGGTCTCGCAGCGTTCACTGGTTCCTCCATCAGCAGCA
AAGTTAAAATCGCCGATCAGTCGATCCTTAACAGCAATC ATG GTT AAG TCA ATT CTT GCA TCC GTT CTC

TTT GCA GCG ACC GCG CTG GCC GCG AGC CGC ATG ACG GCT CCT TCC GGT GCG ATT GTT GTT

GCC AAG TCC GGA GGT GAC TAC GAC ACG gtaagcatctccattgtcgatcgcatgttgcagatgtgtggct
-----------------INTRON 1-----------------
gatagtgtttgcttggacag ATC AGC GCT GCC GTT GAT GCT CTC AGC ACG ACT TCG ACC GAG
--------------------
ACC CAG ACC ATC TTC ATT GAG GAG GGA TCC TAC GAC GAG CAG GTG TAT ATT CCC GCC CTC

AGT GGA AAG CTG ATT GTC TAC GGT CAG ACT GAG GA gtacgtttgtcgttgcccttacgaagtcgacg
------INTRON 2-----------------
atactaaattatccttag C ACT ACC ACC TAC ACC AGC AAC CTG GTC AAC ATC ACC CAC GCC
------------------
ATC GCT TTG GCC GAT GTC GAC AAT GAC GAT GAG ACT G gtcagtctgccagactggaagatattcct
---INTRON 3-----------------
gatttcgctaacgatacgcag CA ACC CTC CGT AAC TAC GCT GAA GGC TCG GCC ATC TAC AAC
---------------------
CTC AAC ATT GCC AAC ACC TGC GGT CAG GCC TGC CAC CAG GCT CTC GCC GTG AGC GCC TAT

GCC AGC GAG CAG GGA TAC TAC GCC TGC CAG TTC ACC GGA TAC CAG G gtagggccaaatccaga
------INTRON 4---
tatgacaatctacagtgactaataccagtatag AC ACC CTT CTG GCT GAG ACC GGC TAC CAG GTT
---------------------------------
TAC GCC GGA ACC TAC ATC GAG GGT GCC GT gtatgtctcccatcataccttcaaaatggtgaacacagct
---------------INTRON 5-----------------
aacataatcaccag C GAC TTC ATC TTT GGA CAG CAC GCC CGC GCC TGG TTC CAC GAG TGC
--------------
GAC ATC CGC GTC CTC GAG GGC CCC AGC TCC GCC TCC ATC ACC GCC AAC GGC CGC TCC TCC

GAG TCG GAC GAC TCT TAC TAC GTG ATC CAC AAG TCC ACC GTC GCT GCT GCT GAT GGC AAC

GAT GTT TCC TCC GGC ACC TAC TAC CTC G gtatgccactagcctagattcgctcaaaagctactagccc
---------------INTRON 6-----------------
actaacaaatatttag GC CGC CCC TGG TCC CAG TAC GCT CGC GTC TGC TTC CAG AAG ACC
-----------------
TCC ATG ACC GAT GTG ATC AAC CAC CTC GGC TGG ACT GAG TGG TCG ACC TCC ACC CCC AAC

ACC GAG AAC GTC ACC TTT GTT GAA TAC GGC AAC ACC GGC ACT GGC GCT GAG GGT CCC CGT

GCT AAC T[?]C TCT TCT GAG CTG ACT GAG CCC ATC ACT ATC TCT TGG CTT CTC GGA TCT GAC

TGG GAG GAC TGG GTT GAT ACT AGC TAC ATC AAC TAA ACTTGTGTGAAAGTTCTAAGCTGGTTGGAGA
AGGGTCGTATATAGTGTTGTGGATCTGTATATATGTGAGATGGAGATATAGCTAATTGTGGTTAGTTAATCACTGCTCA
ATTCATTTCGAAGCAACTTTTATTGGTGGACTTTTGTGCCGTGATTGGGAAAGGACTAGACTGCAGTTGGAGTAATG
CAGACTATCACGAATAAAGTTAGCGTGTGTATTCGCAATGAGCTAACTAGTAACCACTCCTAATATGGAGTAGCACAGC
ATAACTCTGCATCCTGCCACACACACTATCTTGTTCTTCATAAATCGCGTACTATTTACTCTCCATTCAATCCACCTTC
TCATATACTCTATACACCCACCCAACTGGCCCGATAAACTAATATACCATGCCTACTCATACCGAACCTTATTAATCAA
CGTCCCAACACCCCCAATCCTAACCCGAATATCATCCCCATCCCTGAGCACCACCCTCGGCTCTTTAAAGAAGCCAATT
CCCGCCGGCGTCCCGGTAAGAATAATAGTCCCGGCCTCCAACGTCGTACCTTGTGACAGGTACGCGATCGTCTTCTTC
 or a nucleotide sequence which encodes for the same amino acid sequence but which differs from the indicated nucleotide sequence due to the degeneracy of the genetic code, is used for the production of the recombinant host organism.

9. A method according to at least one of claims 5 to 8, characterised in that the deoxyribonucleic acid originates from Aspergillus niger DSM 5575.

10. A method according to claim 9, characterised in that the deoxyribonucleic acid encodes polygalacturonase from Aspergillus niger DSM 5575.

11. A method according to claim 9, characterised in that the deoxyribonucleic acid encodes pectinesterase from Aspergillus niger DSM 5575.

12. A method according to at least one of claims 5 to 11, characterised in that the fungus strains Aspergillus niger DSM 5575 or Aspergillus awamori 5574 are used as the host organism.

13. A method according to at least one of claims 5 to 12, characterised in that the pectinase gene is multiply-inserted into the genome of the host organism.

14. A method according to at least one of claims 5 to 13, characterised in that a deoxyribonucleic acid is used which encodes a macerating polygalacturonase.

15. A method of producing polygalacturonase or pectinesterase, characterised in that the recombinant host organism obtained according to at least one of claims 5 to 14 is propagated in a suitable nutrient medium and the polygalacturonase or pectinesterase is isolated in a manner known in the art.

## Revendications

1. Acide désoxyribonucléique *d'Aspergillus niger* dont la séquence nucléotidique est codante pour une polygalacturonase ou une pectine-estérase, caractérisé en ce que la polygalacturonase possède la séquence nucléotidique suivante 
GGA AGC TGC ACC TTC AAA ACG GCT GCT GCT GCC AAA GCG GGC AAG GCA GGG TGC TCT ACC
ATC ACC CTT GAC AAC ATC GAA GTC CCC GCT GGA ACC ACC CTC GAC CTG ACC GGT CTC ACC
AGC GGT ACG AAG GTC ATC TTC GAG GGC ACC ACG ACC TTC GAT TAT GAA GAA TGG GCA GGG
CCC TTG ATC TCC ATG AGT GGC AAA GAT ATC ACC GTC ACT GGT GCC TCA GGC CAT CTC ATC
AAC TGC GAC GGT GCG CGG TGG TGG GAC GGC AAG GGG ACC AGC GGA AAG AAG AAG CCC AAG
TTC TTC TAC GCT CAT GGC CTT GAC TCC TCG TCC ATT ACT GGA TTG AAT ATC AAG AAC ACT
CCC CTT ATG GCG TTT AGT GTT CAG GCG GAT GAC ATC ACT CTG ACT GAC ATT ACC ATC AAC
AAC GCG GAC GGT GAT ACC CTG GGT GGA CAC AAC ACT GAT GCG TTT GAT GTT GGT AAC TCT
GTC GGT GTG AAT ATC ATC AAA CCG TGG GTC CAT AAC CAG GAT GAC TGT CTT GCG ATC AAC
TCT GGC GAG AAC ATC TGG TTT ACC AGC GGC ACC TGC ATT GGC GGC CAC GGT CTC TCC ATC
GGT TCT GTC GGC GGC CGC TCC AAC AAC GTT GTC AAG AAC GTC ACT ATC GAA CAC TCC ACC
GTG AGC AAT TCC GAG AAC GCC GTC CGG ATT AAG ACC GTC TCT GGT GCC ACT GGT TCC GTG
TCT GAG ATC ACA TAC TCC AAC ATT GTC ATG TCC GGC ATC TCC GAT TAC GGC GTC GTT ATC
CAG CAG GAT TAC GAG GAT GGC AAG CCT ACG GGT AAG CCC ACG AAC GGT GTC ACT ATT ACG
GAT GTC AAG CTG GAG AGC GTG ACT GGT ACT GTG GAT AGT AAG GCT ACT GAT ATC TAT CTC
CTT TGC GGA TCT GGT AGC TGC TCG GAC TGG ACT TGG GAC GAT GTG AAG GTC ACT GGA GGA
AAG AAG TCT ACT GCT TGC AAA AAG TAC CCT TCG GTG GCT TCT TGC
 ou une séquence nucléotidique qui est codante pour la même séquence d'amino-acides, mais qui se différencie de la séquence nucléotidique indiquée sur la base de la dégénération du code génétique, et la pectine-estérase possède la séquence nucléotidique suivante 
GCG AGC CGC ATG ACG GCT CCT TCC GGT GCG ATT GTT GTT GCC AAG TCC GGA
GGT GAC TAC GAC ACG ATC AGC GCT GCC GTT GAT GCT CTC AGC ACG ACT TCG
ACC GAG ACC CAG ACC ATC TTC ATT GAG GAG GGA TCC TAC GAC GAG CAG GTG
TAT ATT CCC GCC CTC AGT GGA AAG CTG ATT GTC TAC GGT CAG ACT GAG GAC
ACT ACC ACC TAC ACC AGC AAC CTG GTC AAC ATC ACC CAC GCC ATC GCT TTG
GCC GAT GTC GAC AAT GAC GAT GAG ACT GCA ACC CTC CGT AAC TAC GCT GAA
GGC TCG GCC ATC TAC AAC CTC AAC ATT GCC AAC ACC TGC GGT CAG GCC TGC
CAC CAG GCT CTC GCC GTG AGC GCC TAT GCC AGC GAG CAG GGA TAC TAC GCC
 
TGC CAG TTC ACC GGA TAC CAG GAC ACC CTT CTG GCT GAG ACC GGC TAC CAG
GTT TAC GCC GGA ACC TAC ATC GAG GGT GCC GTC GAC TTC ATC TTT GGA CAG
CAC GCC CGC GCC TGG TTC CAC GAG TGC GAC ATC CGC GTC CTC GAG GGC CCC
AGC TCC GCC TCC ATC ACC GCC AAC GGC CGC TCC TCC GAG TCG GAC GAC TCT
TAC TAC GTG ATC CAC AAG TCC ACC GTC GCT GCT GCT GAT GGC AAC GAT GTT
TCC TCC GGC ACC TAC TAC CTC GGC CGC CCC TGG TCC CAG TAC GCT CGC GTC
TGC TTC CAG AAG ACC TCC ATG ACC GAT GTG ATC AAC CAC CTC GGC TGG ACT
GAG TGG TCG ACC TCC ACC CCC AAC ACC GAG AAC GTC ACC TTT GTT GAA TAC
GGC AAC ACC GGC ACT GGC GCT GAG GGT CCC CGT GCT AAC TTC TCT TCT GAG
CTG ACT GAG CCC ATC ACT ATC TCT TGG CTT CTC GGA TCT GAC TGG GAG GAC
TGG GTT GAT ACT AGC TAC ATC AAC
 ou une séquence nucléotidique qui est codante pour la même séquence d'amino-acides, mais qui se différencie de la séquence nucléotidique indiquée sur la base de la dégénération du code génétique.

2. Acide désoxyribonucléique selon la revendication 1, caractérisé en ce qu'il provient *d'Aspergillus niger* DSM 5575.

3. Acide désoxyribonucléique selon la revendication 1 ou 2, caractérisé en ce qu'il est codant pour la polygalacturonase *d'Aspergillus niger* DSM 5575.

4. Acide désoxyribonucléique selon la revendication 1, caractérisé en ce qu'il est codant pour la pectine-estérase *d'Aspergillus niger* DSM 5575.

5. Procédé de préparation d'un organisme hôte recombinant qui est transformé avec un gène de polygalacturonase ou avec un gène de pectine-estérase *d'Aspergillus,* par clonage d'un vecteur recombinant qui contient le gène de polygalacturonase ou le gène de pectine-estérase, ainsi que les séquences encadrantes de désoxyribonucléotides nécessaires à leur expression dans *Aspergillus,* et transformation du vecteur recombinant dans un organisme hôte, caractérisé en ce qu'on utilise, comme gène de polygalacturonase ou comme gène de pectine-estérase, un acide désoxyribonucléique *d'Aspergillus niger* selon l'une des revendications 1 à 4 ou un acide désoxyribonucléique chromosomique correspondant, et qu'on utilise, comme organisme hôte, un hyphomycète de l'espèce *Aspergillus niger ou Aspergillus awamori.*

6. Procédé selon la revendication 5, caractérisé en ce qu'on effectue les étapes suivantes :
(a) mise en culture *d'Aspergillus niger* dans des conditions d'induction de polygalacturonase ou de pectine-estérase,
(b) purification de la polygalacturonase ou de la pectine-estérase et séquençage partiel des protéines,
(c) isolement des acides ribonucléiques messagers à partir des cellules cultivées en (a),
(d) production d'une banque d'acide désoxyribonucléique-copie à partir des acides ribonucléiques obtenus en (c),
(e) préparation d'une sonde d'oligodésoxyribonucléotides qu'on obtient à partir de la séquence protéique obtenue en (b),
(f) clonage de l'acide désoxyribonucléique-copie contenant le gène de polygalacturonase ou le gène de pectine-estérase en utilisant la sonde d'oligo-désoxyribonucléotides obtenue en (e), comme sonde d'hybridation,
(g) production d'une banque de gènes qui contient l'acide désoxyribonucléique chromosomique de la souche *d'Aspergillus* utilisée en (a),
(h) clonage d'un vecteur recombinant à partir de la banque de gènes selon (g), qui contient le gène chromosomique de polygalacturonase ou le gène de pectine-estérase, ainsi que les séquences encadrantes de désoxyribonucléotides nécessaires à l'expression dans *Aspergillus,* en utilisant l'acide désoxyribonucléique-copie provenant de (f), comme sonde d'hybridation,
(i) clonage du gène de polygalacturonase ou du gène de pectine-estérase ainsi que des séquences encadrantes de désoxyribonucléotides nécessaires à l'expression dans *Aspergillus,* dans un plasmide,
(k) cotransformation d'un organisme hôte *d'Aspergillus niger* ou *d'Aspergillus awamori* au moyen du vecteur recombinant selon (i) et d'un deuxième plasmide qui contient un marqueur de sélection pouvant être exprimé dans l'organisme hôte,
(1) sélection d'une souche cotransformée obtenue en (k) dont le rendement en polygalacturonase ou en pectine-estérase est supérieur à celui de *l'Aspergillus niger* utilisé en (a).

7. Procédé selon la revendication 5 ou 6, caractérisé en ce que, pour la préparation de l'organisme hôte recombinant, on utilise un acide désoxyribonucléique chromosomique qui est codant pour la polygalacturonase *d'Aspergillus niger* et possède la séquence nucléotidique suivante 
AAGCTTTCAACCCTGATTAGCGAGCTTTGCACACTATTCCCGATGTAGCTGTCGAAGGAACGGAT
GGACGCTGCACTAGTTTCATTTCGAATCTCTGATATTAATACTAACTACAGAGTGACTA
GCCGATCACGCTCCGGATCTGGCACAGAGTGATTTTTTGAATCATTCATTGGTGGAATT
TGCAAGGCACCATAAATGCCGCAGACCCTGCATATCGAGCTACAGCCTTACTGGGATTA
GTATAGCTGCCATCTCGTACTCGTACACTGCAGAACCGGCTTGGATCCCAAGGCCTACG
ATGACACCCGTTTCCTCCAGGACTGATGGCTGTTTGTGAAGGCGAATTATAGTACTGCT
TGTCAATGTCTTGAGTCAGCGGTATATATGAATCTGTCTTTGTCTTTCCACCTTGACAA
TAATGTTACGCTCAAGAGGGCATTTTGCGTTCTTTTCATCGACATGCGAACCTGTTCGG
TCACCCGCGGACCCCGTCGGCTGATCAGCCACCACGGCTCATATATATTAGTTGCCCAC
CATGTCGAACTTAAGTTCATTAAAAAAAAGGTAACATTTGAGACAATATCTTAATGTGA
AACGTGAACCCTGGACTAGCATCTCTCCAGAGGCTGTCGGCAGTTATGACTTTCCGATC
AGAAGAGATGCGCTGAAATTGTGACTATAAGAACCTCAAGCCTGCCGATGCTGAGGTGA
GTTTGCTCATCATCCTACACTCATTTGGCATCAGACCGATTACACTCTTTTTGTCCTTT
TTTTCTATCGCTATCATTGACC ATG CAC TCC TTT GCT TCT CTT CTG GCC
TAC GGC CTA GCC GCC AGC GCC ACC CTC GCT TCT GCC TCC CCC ATC
GAA GCC CGG GGA AGC TGC ACC TTC AAA ACG GCT GCT GCT GCC AAA
GCG GGC AAG GCA GGG TGC TCT ACC ATC ACC CTT GAC AAC ATC GAA
GTC CCC GCT GGA ACC ACC CTC GAC CTG ACC GGT CTC ACC AGC GGT
ACG AAG GTC ATC TTC GAG GGC ACC ACG ACC TTC GAT TAT GAA GAA
TGG GCA GGG CCC TTG ATC TCC ATG AGT GGC AAA GAT ATC ACC GTC
ACT GGT GCC TCA GGC CAT CTC ATC AAC TGC GAC GGT GCG CGG TGG
TGG GAC GGC AAG GGG ACC AGC GGA AAG AAG AAG CCC AAG TTC TTC
TAC GCT CAT GGC CTT GAC TCC TCG TCC ATT ACT GGA TTG AAT ATC
 
AAG AAC ACT CCC CTT ATG GCG TTT AGT GTT CAG GCG GAT GAC ATC
ACT CTG ACT GAC ATT ACC ATC AAC AAC GCG GAC GGT GAT ACC CTG
GGT GGA CAC AAC ACT GAT GCG TTT GAT GTT GGT AAC TCT GTC GGT
GTG AAT ATC ATC AAA CCG TGG GTC CAT AAC CAG GAT GAC TGT CTT
GCG ATC AAC TCT GGC GAG gtaagcagctttgaacatagatttgatttgcatg
<---------------INTRON 1----------
tatgttgatattctatag AAC ATC TGG TTT ACC AGC GGC ACC TGC ATT
----------------->
GGC GGC CAC GGT CTC TCC ATC GGT TCT GTC GGC GGC CGC TCC AAC
AAC GTT GTC AAG AAC GTC ACT ATC GAA CAC TCC ACC GTG AGC AAT
TCC GAG AAC GCC GTC CGG ATT AAG ACC GTC TCT GGT GCC ACT GGT
TCC GTG TCT GAG ATC ACA TAC TCC AAC ATT GTC ATG TCC GGC ATC
TCC GAT TAC GGC GTC GTT ATC CAG CAG GAT TAC GAG GAT GGC AAG
CCT ACG GGT AAG CCC ACG AAC GGT GTC ACT ATT ACG GAT GTC AAG
CTG GAG AGC GTG ACT GGT ACT GTG GAT AGT AAG GCT ACT GAT ATC
TAT CTC CTT TGC GGA TCT GGT AGC TGC TCG GAC TGG ACT TGG GAC
GAT GTG AAG GTC ACT GGA GGA AAG AAG TCT ACT GCT TGC AAA AAC
TAC CCT TCG GTG GCT TCT TGC TAG GTTAGTAGGTTGTTCGGTTGTAGCAC
TTGCTAACATGCATTTGCCTTGAGGGGTCAAATGGATTTGTGAAATTATCGGTGGAAAG
AAGAGATGGTGTCCAGTAAGATTCAGTGGTGGC
 ou une séquence nucléotidique qui est codante pour la même séquence d'amino-acides, mais qui se différencie de la séquence nucléotidique indiquée sur la base de la dégénération du code génétique.

8. Procédé selon la revendication 5 ou 6, caractérisé en ce que, pour la préparation de l'organisme hôte recombinant, on utilise un acide désoxyribonucléique chromosomique qui est codant pour la pectine-estérase *d'Aspergillus niger* et possède la séquence nucléotidique suivante 
AAGCTTCTTTGAACGCCCCCACATTGTCAACGGCCGGCCCAATTGCCTGACTTCTCCTAGTCTTCTCAGATATGGTTGT
TCAAGCTGTGACAGAACCCCGCCGGCGTCTCCACACGGAGGTCCTGAACCTTATGAGGCAAATACTGACCGATAACAAG
AGCTCATGATGAGTGGATGGCATGCGGTTCCTATAAGAACGAGGTCTCGCAGCGTTCACTGGTTCCTCCATCAGCAGCA
AAGTTAAAATCGCCGATCAGTCGATCCTTAACAGCAATC ATG GTT AAG TCA ATT CTT GCA TCC GTT CTC

TTT GCA GCG ACC GCG CTG GCC GCG AGC CGC ATG ACG GCT CCT TCC GGT GCG ATT GTT GTT

GCC AAG TCC GGA GGT GAC TAC GAC ACG gtaagcatctccattgtcgatcgcatgttgcagatgtgtggct
-----------------INTRON 1-----------------
gatagtgtttgcttggacag ATC AGC GCT GCC GTT GAT GCT CTC AGC ACG ACT TCG ACC GAG
--------------------
ACC CAG ACC ATC TTC ATT GAG GAG GGA TCC TAC GAC GAG CAG GTG TAT ATT CCC GCC CTC

AGT GGA AAG CTG ATT GTC TAC GGT CAG ACT GAG GA gtacgtttgtcgttgcccttacgaagtcgacg
------INTRON 2------------------
atactaaattatccttag C ACT ACC ACC TAC ACC AGC AAC CTG GTC AAC ATC ACC CAC GCC
------------------
 
ATC GCT TTG GCC GAT GTC GAC AAT GAC GAT GAG ACT G gtcagtctgccagactggaagatattcct
---INTRON 3------------------
gatttcgctaacgatacgcag CA ACC CTC CGT AAC TAC GCT GAA GGC TCG GCC ATC TAC AAC
---------------------
CTC AAC ATT GCC AAC ACC TGC GGT CAG GCC TGC CAC CAG GCT CTC GCC GTG AGC GCC TAT

GCC AGC GAG CAG GGA TAC TAC GCC TGC CAG TTC ACC GGA TAC CAG G gtagggccaaatccaga
------INTRON 4---
tatgacaatctacagtgactaataccagtatag AC ACC CTT CTG GCT GAG ACC GGC TAC CAG GTT
---------------------------------
TAC GCC GGA ACC TAC ATC GAG GGT GCC GT gtatgtctcccatcataccttcaaatggtgaacacagct
---------------INTRON 5------------------
aacataatcaccag C GAC TTC ATC TTT GGA CAG CAC GCC CGC GCC TGG TTC CAC GAG TGC
--------------
GAC ATC CGC GTC CTC GAG GGC CCC AGC TCC GCC TCC ATC ACC GCC AAC GGC CGC TCC TCC

GAG TCG GAC GAC TCT TAC TAC GTG ATC CAC AAG TCC ACC GTC GCT GCT GCT GAT GGC AAC

GAT GTT TCC TCC GGC ACC TAC TAC CTC G gtatgccactagcctagattcgctcaaaagctactagccc
---------------INTRON 6------------------
actaacaaatattttag GC CGC CCC TGG TCC CAG TAC GCT CGC GTC TGC TTC CAG AAG ACC
-----------------
TCC ATG ACC GAT GTG ATC AAC CAC CTC GGC TGG ACT GAG TGG TCG ACC TCC ACC CCC AAC

ACC GAG AAC GTC ACC TTT GTT GAA TAC GGC AAC ACC GGC ACT GGC GCT GAG GGT CCC CGT

GCT AAC T[?]C TCT TCT GAG CTG ACT GAG CCC ATC ACT ATC TCT TGG CTT CTC GGA TCT GAC

TGG GAG GAC TGG GTT GAT ACT AGC TAC ATC AAC TAA ACTTGTGTGAAAGTTGTAAGCTGGTTGGAGA
AGGGTCGTATATAGTGTTGTGGATCTGTATATATGTGAGATGGAGATATAGCTAATTGTGGTTAGTTAATCACTGCTCA
ATTCATTTCGAAGCAACTTTTATTGGTGGACTTTTGTGCCGTGATTGGGAAAGGGACTAGACTGCAGTTGGAGTAATG
CAGACTATCACGAATAAAGTTAGCGTGTGTATTCGCAATGAGCTAACTAGTAACCACTCCTAATATGGAGTAGCACAGC
ATAACTCTGCATCCTGCCACACACACTATCTTGTTCTTCATAAATCGCGTACTATTTACTCTCCATTCAATCCACCTTC
TCATATACTCTATACACCCACCCAACTGGCCCGATAAACTAATATACCATGCCTACTCATACCGAACCTTATTAATCAA
CGTCCCAACACCCCCAATCCTAACCCGAATATCATCCCCATCCCTGAGCACCACCCTCGGCTCTTTAAAGAAGCCAATT
CCCGCCGGCGTCCCGGTAAGAATAATAGTCCCGGCCTCCAACGTCGTACCTTGTGACAGGTACGCGATCGTCTTCTTC
 ou une séquence nucléotidique qui est codante pour la même séquence d'amino-acides, mais qui se différencie de la séquence indiquée sur la base de la dégénération du code génétique.

9. Procédé selon au moins une des revendications 5 à 8, caractérisé en ce que l'acide désoxyribonucléique provient *d'Aspergillus niger* DSM 5575.

10. Procédé selon la revendication 9, caractérisé en ce que l'acide désoxyribonucléique est codant pour la polygalacturonase *d'Aspergillus niger* DSM 5575.

11. Procédé selon la revendication 9, caractérisé en ce que l'acide désoxyribonucléique est codant pour la pectine-estérase *d'Aspergillus niger* DSM 5575.

12. Procédé selon au moins une des revendications 5 à 11, caractérisé en ce qu'on utilise, comme organisme hôte, les souches d'hyphomycètes *Aspergillus niger* DSM 5575 ou *Aspergillus awamori* 5574.

13. Procédé selon au moins une des revendications 5 à 12, caractérisé en ce que le gène de pectinase est inséré plusieurs fois dans le génome de l'organisme hôte.

14. Procédé selon au moins une des revendications 5 à 13, caractérisé en ce qu'on utilise un acide désoxyribonucléique qui est codant pour une polygalacturonase en macération.

15. Procécé de préparation d'une polygalacturonase ou d'une pectine-estérase, caractérisé en ce que l'organisme hôte recombinant obtenu selon au moins une des revendications 5 à 14 s'accroît dans un milieu nutritif approprié, et on isole la polygalacturonase ou la pectine-estérase d'une manière connue en soi.
